(19) Europäisches Patentamt
European Patent Office
Office européen des brevets

(11) EP 3 650 447 A1

(12) EUROPEAN PATENT APPLICATION

(43) Date of publication:
**13.05.2020 Bulletin 2020/20**

(51) Int Cl.:
*C07D 401/12* (2006.01) *A01N 43/36* (2006.01)
*C07D 207/48* (2006.01) *A61P 31/04* (2006.01)

(21) Application number: **18306468.2**

(22) Date of filing: **08.11.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Universite de Nantes**
  **44000 Nantes (FR)**
- **CENTRE NATIONAL DE LA RECHERCHE SCIENTIFIQUE**
  **75016 Paris (FR)**
- **Université d'Orléans**
  **45100 Orléans (FR)**

(72) Inventors:
- **LE QUESTEL, Jean-Yves**
  **44115 HAUTE GOULAINE (FR)**
- **GRATON, Jérôme**
  **44330 LE PALLET (FR)**
- **LAURENT, Adèle**
  **44300 NANTES (FR)**
- **SELVAM, Balaji**
  **URBANA, 61801 (US)**
- **LEBRETON, Jacques**
  **44300 NANTES (FR)**
- **MATHE-ALLAINMAT, Monique**
  **44300 NANTES (FR)**
- **LANDAGARAY, Elodie**
  **64640 ARMENDARITS (FR)**
- **THANY, Steeve**
  **45240 MENESTREAU EN VILLETTE (FR)**
- **CARTEREAU, Alison**
  **45160 OLIVET (FR)**

(74) Representative: **Lavoix**
**2, place d'Estienne d'Orves**
**75441 Paris Cedex 09 (FR)**

# (54) NEW SELECTIVE MODULATORS OF INSECT NICOTINIC ACETYLCHOLINE RECEPTORS

(57) The present invention relates to a compound having the following formula (I):

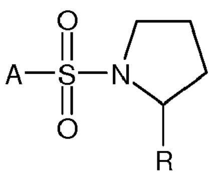

(I)

wherein:
- A is a (hetero)aryl radical comprising from 5 to 10 carbon atoms, possibly substituted by at least one substituent chosen from the group consisting of: halogen atoms, amino, azido, cyano, nitro, hydroxyl, formyl, carboxyl, amido, ($C_1$-$C_6$)alkyl groups, halo($C_1$-$C_6$)alkyl groups, ($C_1$-$C_6$)alkoxy groups, alkenyl groups, cycloalkenyl groups, and alkynyl groups, and
- R is H, CN or $CF_3$,

or their pharmaceutically acceptable salts, racemates, diastereomers or enantiomers.

EP 3 650 447 A1

## Description

The present invention concerns new competitive modulators of insect nicotinic acetylcholine receptors, as well as uses thereof especially as insecticides.

The resistance of important insect pests to a broad range of insect control agents, in particular to neonicotinoids, which have had a leading position on the global insecticide sales (more than 25% in 2014) has brought insecticides chemists to develop new insect control tools (for example, sulfoxaflor (SFX), the representative of sulfoximine (Sparks, T.C.; Watson, G.B.; Loso, M.R.; Geng, C.; Babcock, J.M.; Thomas, J.D., Sulfoxaflor and the sulfoximine insecticides: Chemistry, mode of action and basis for efficacy on resistant insects. Pesticide Biochemistry and Physiology 2013, 107, (1), 1-7), and flupyradifurone, member of the butenolide sub-class (Nauen, R.; Jeschke, P.; Velten, R.; Beck, M.E.; Ebbinghaus-Kintscher, U.; Thielert, W.; Woelfel, K.; Haas, M.; Kunz, K.; Raupach, G., Flupyradifurone: a brief profile of a new butenolide insecticide. Pest Management Science 2015, 71, (6), 850-862)). These compounds, targeting nicotinic Acetylcholine receptors (nAChRs), represent new subgroups of the main group 4 of the IRAC classification, corresponding to insect nAChRs competitive modulators. Thus, sulfoximine and butenolide correspond respectively to 4C and 4D subgroups.

Despite their distinct chemical features with respect to neonicotinoids, the question of the harmlessness of these new insect nAChR competitive modulators towards pollinators and their predators remains a highly controversial subject (Supuran, C. T.; Innocenti, A.; Mastrolorenzo, A.; Scozzafava, A., Antiviral sulfonamide derivatives. Mini-Reviews in Medicinal Chemistry 2004, 4, (2), 189-200; Campbell, J. W.; Cabrera, A. R.; Stanley-Stahr, C.; Ellis, J. D., An Evaluation of the Honey Bee (Hymenoptera: Apidae) Safety Profile of a New Systemic Insecticide, Flupyradifurone, Under Field Conditions in Florida. Journal of Economic Entomology 2016, 109, (5), 1967-1972; and Scozzafava, A.; Owa, T.; Mastrolorenzo, A.; Supuran, C. T., Anticancer and antiviral sulfonamides. Current Medicinal Chemistry 2003, 10, (11), 925-953).

Thus, there remains a need of alternative tools for pest management, especially without the drawbacks of the current tools.

The aim of the present invention in this context is to provide new inhibitors of insect nicotinic acetylcholine receptors.

Another aim of the present invention is to provide new useful pesticides harmless for non-target insects like pollinators such as honey bees.

Thus, the present invention relates to compounds having the following formula (I):

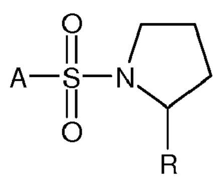

(I)

wherein:

- A is a (hetero)aryl radical comprising from 5 to 10 carbon atoms, possibly substituted by at least one substituent chosen from the group consisting of: halogen atoms, amino ($-NH_2$), azido ($N_3$), cyano (CN), nitro ($-NO_2$), hydroxyl (-OH), formyl (-C(O)H), carboxyl (-COOH), amido ($-C(O)-NH_2$), ($C_1$-$C_6$)alkyl groups, halo($C_1$-$C_6$)alkyl groups, ($C_1$-$C_6$)alkoxy groups, alkenyl groups, cycloalkenyl groups, and alkynyl groups, and
- R is H, CN or $CF_3$, and preferably H or CN,

or their pharmaceutically acceptable salts, racemates, diastereomers or enantiomers.

The compounds of formula (I) can comprise one or more asymmetric carbon atoms. They can therefore exist in the form of enantiomers or of diastereoisomers. These enantiomers and diastereoisomers, and also mixtures thereof, including racemic mixtures, form part of the invention.

The compounds of formula (I) can exist in the form of bases or of addition salts with acids. Such addition salts form part of the invention.

The compounds of formula (I) can exist in the form of pharmaceutically acceptable salts. These salts can be prepared with pharmaceutically acceptable acids, but the salts of other acids that are of use, for example, for purifying or isolating the compounds of formula (I) also form part of the invention.

In the above formula (I), A is a (hetero)aryl radical which means that A may be either an aryl radical or a heteroaryl radical.

According to the invention, the term "aryl group" means a cyclic aromatic group comprising between 6 and 10

carbon atoms. By way of examples of aryl groups, mention may be made of phenyl or naphthyl groups. According to an embodiment, A is a phenyl group.

According to the invention, the term "heteroaryl" means a 5- to 10-membered aromatic monocyclic or bicyclic group containing from 1 to 4 heteroatoms selected from O, S or N.

By way of examples, mention may be made of imidazolyl, thiazolyl, oxazolyl, furanyl, thiophenyl, pyrazolyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl and isoquinolinyl groups.

By way of a heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of heteroaryl, of thiophenyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothiophenyl, thienopyridyl, thienopyrimidinyl, pyrrolopyridyl, imidazopyridyl, benzoazaindole, 1,2,4-triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenyl nucleus.

According to an embodiment, A is an aryl radical comprising from 6 to 10 carbon atoms. Preferably, in formula (I), A is a phenyl group.

According to another embodiment, A is a heteroaryl radical comprising from 5 to 10 atoms, and including at least one heteroatom. Preferably, in formula (I), A is a pyridinyl group.

A preferred group of compounds according to the invention consists in compounds of formula (I) wherein A is a phenyl group and R is H, CN or $CF_3$, and preferably H or CN, A being possibly substituted as mentioned above.

A preferred group of compounds according to the invention consists in compounds of formula (I) wherein A is a pyridinyl group and R is H, CN or $CF_3$, and preferably H or CN, A being possibly substituted as mentioned above.

As mentioned above, A may be substituted by at least one substituent. In other words, the abovementioned "aryl" and "heteroaryl" radicals A can be substituted with one or more substituents. Among these substituents, mention may be made of the following groups: halogen atoms, amino ($-NH_2$), azido ($N_3$), cyano (CN), nitro ($-NO_2$), hydroxyl (-OH), formyl (-C(O)H), carboxyl (-COOH), amido ($-C(O)-NH_2$), $-SO_3H$, $-PO_3H_2$, $(C_1-C_6)$alkyl groups, halo$(C_1-C_6)$alkyl groups, $(C_1-C_6)$alkoxy groups, alkylamino groups, alkenyl groups, cycloalkenyl groups, and alkynyl groups.

As substituents, the followings may also be mentioned: $-C(O)R_a$, $-COOR_a$, $-SO_3R_a$, $-PO_3R_aR_b$, $-NR_aR_b$ and $-C(O)-NR_aR_b$, wherein $R_a$ and $R_b$ are, independently from each other, $(C_1-C_6)$alkyl groups.

In the context of the present invention, the expression "$C_t-C_z$" means a carbon-based chain which can have from t to z carbon atoms, for example $C_1-C_3$ means a carbon-based chain which can have from 1 to 3 carbon atoms.

Within the present application, the term "a halogen atom" means: a fluorine, a chlorine, a bromine or an iodine.

Within the present invention, the term "an alkyl group" means: a linear or branched, saturated, hydrocarbon-based aliphatic group comprising, unless otherwise mentioned, from 1 to 6 carbon atoms. By way of examples, mention may be made of methyl, ethyl, n-propyl, isopropyl, butyl, isobutyl, tert-butyl or pentyl groups.

According to the invention, the term "a haloalkyl group" means: an alkyl group as defined above, in which one or more of the hydrogen atoms is (are) replaced with a halogen atom. By way of example, mention may be made of fluoroalkyls, in particular $CF_3$ or $CHF_2$.

According to the invention, the term "an alkoxy group" means: an -O-alkyl radical where the alkyl group is as previously defined. By way of examples, mention may be made of $-O-(C_1-C_4)$alkyl groups, and in particular the -O-methyl group, the -O-ethyl group as $-O-C_3$alkyl group, the -O-propyl group, the -O-isopropyl group, and as $-O-C_4$alkyl group, the -O-butyl, -O-isobutyl or -O-tert-butyl group.

According to the invention, the term "an alkylamino" means an -NH-alkyl group, the alkyl group being as defined above.

According to the invention, the term "alkenyl" refers to acyclic branched or unbranched hydrocarbons having a carbon-carbon double bond and the general formula $C_nH_{2n-1}$, comprising, unless otherwise mentioned, from 2 to 6 carbon atoms.

According to the invention, the term "cycloalkenyl" refers to a cyclic alkenyl, said alkenyl group being as defined above.

According to the invention, the term "alkynyl" refers to acyclic branched or unbranched hydrocarbons having a carbon-carbon triple bond and the general formula $C_nH_{2n-2}$, comprising, unless otherwise mentioned, from 2 to 6 carbon atoms.

According to an embodiment, the compounds of the invention have the formula (I) as defined above, wherein A is a phenyl group, possibly substituted by at least one substituent as defined above. Preferably, A is substituted by at least one substituent chosen from the group consisting of: halogen atoms, $(C_1-C_6)$alkyl groups, and $(C_1-C_6)$alkoxy groups, preferably Cl, $OCH_3$ or $CH_3$.

According to an embodiment, the compounds of the invention have the formula (I) as defined above, wherein

A is a heteroaryl group comprising from 5 to 10 atoms including 1 to 4 heteroatoms selected from O, S or N, possibly substituted by at least one substituent as defined above. Preferably, A is substituted by at least one substituent chosen from the group consisting of: halogen atoms, ($C_1$-$C_6$)alkyl groups, and ($C_1$-$C_6$)alkoxy groups, preferably Cl, $OCH_3$ or $CH_3$.

According to an embodiment, the compounds of the invention have the formula (I) as defined above, wherein A is a heteroaryl group comprising from 5 to 10 atoms including at least one nitrogen atom, possibly substituted by at least one substituent as defined above. Preferably, A is substituted by at least one substituent chosen from the group consisting of: halogen atoms, ($C_1$-$C_6$)alkyl groups, and ($C_1$-$C_6$)alkoxy groups, preferably Cl, $OCH_3$ or $CH_3$.

The present invention also relates to compounds having the following formula (II):

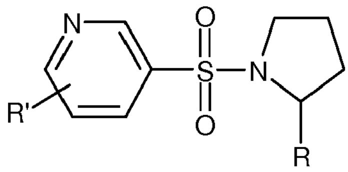

(II)

wherein:

- R is as defined above in formula (I); and
- R' is a substituent chosen from the group consisting of: halogen atoms, amino ($-NH_2$), azido ($N_3$), cyano (CN), nitro ($-NO_2$), hydroxyl (-OH), formyl (-C(O)H), carboxyl (-COOH), amido ($-C(O)-NH_2$), ($C_1$-$C_6$)alkyl groups, halo($C_1$-$C_6$)alkyl groups, ($C_1$-$C_6$)alkoxy groups, alkylamino groups, alkenyl groups, cycloalkenyl groups, and alkynyl groups.

According to an embodiment, in formula (II), R is H or CN.

According to an embodiment, in formula (II), R' is a substituent chosen from the group consisting of: halogen atoms, ($C_1$-$C_6$)alkyl groups, and ($C_1$-$C_6$)alkoxy groups, preferably Cl, $OCH_3$ or $CH_3$.

The present invention also relates to compounds having the following formula (III):

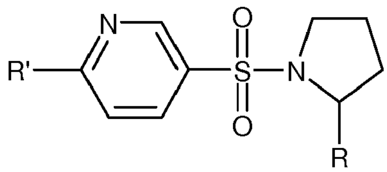

(III)

wherein:

- R is as defined above in formula (I); and
- R' is a substituent chosen from the group consisting of: halogen atoms, ($C_1$-$C_6$)alkyl groups, and ($C_1$-$C_6$)alkoxy groups, preferably Cl, $OCH_3$ or $CH_3$.

According to an embodiment, in formula (III), R is H or CN.

The present invention also relates to the following compounds:

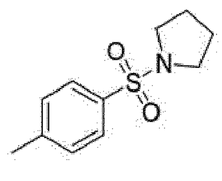
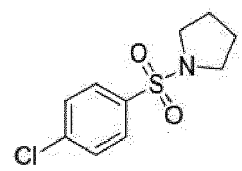

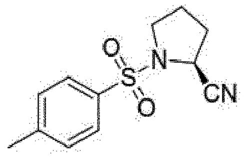
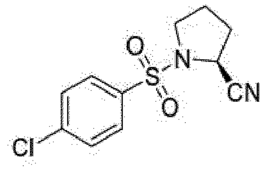

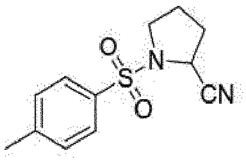
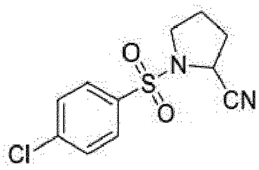

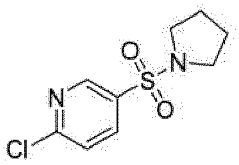
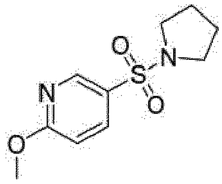

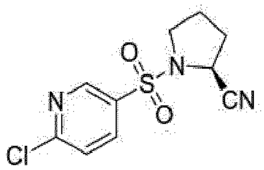
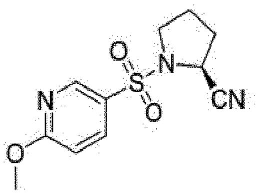
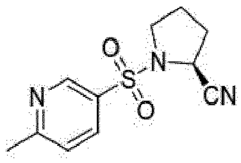

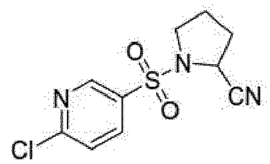
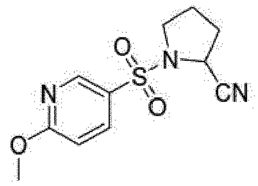

The present invention also relates to a process for preparing the compounds of formula (I) as defined above, comprising the reaction between a compound having the following formula (IV):

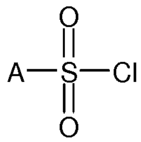
(IV)

A being as defined above in formula (I), with a compound having the following formula (V):

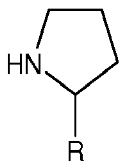
(V)

R being as defined above in formula (I).

According to an embodiment, in formula (IV), A is an aryl radical comprising from 6 to 10 carbon atoms or a heteroaryl radical comprising from 5 to 10 atoms, and including at least one heteroatom. Preferably, in formula (IV), A is a phenyl group or a pyridinyl group, possibly substituted as defined above.

Preferably, in formula (V), R is H or CN.

The compounds of formula (IV), which are (hetero)aromatic sulfonyl chloride compounds, may be prepared by conventional methods well-known from the skilled person.

For example, such compounds may be prepared by direct sulfonylation of aromatic rings in acidic conditions at high temperature with oleum (McElvain, S. M.; Goese, M. A., The Sulfonation of Pyridine and the Picolines. Journal of the American Chemical Society 1943, 65, (11), 2233-2236; Brand, J. C. D., 207. Aromatic sulphonation. Part II. Kinetics of sulphonation in fuming sulphuric acid. Journal of the Chemical Society (Resumed) 1950, (0), 1004-1017; and Thomas,

K.; Jerchel, D., Neuere Methoden der präparativen organischen Chemie II. 12. Die Einführung von Substituenten in den Pyridin-Ring. Angewandte Chemie 1958, 70, (24), 719-737), followed by chlorination with thionyl chloride or phosphorus chloride (Owa, T.; Yoshino, H.; Okauchi, T.; Okabe, T.; Ozawa, Y.; Hata Sugi, N.; Yoshimatsu, K.; Nagasu, T.; Koyanagi, N.; Kitoh, K., Synthesis and biological evaluation of N-(7-indolyl)-3-pyridinesulfonamide derivatives as potent antitumor agents. Bioorganic & Medicinal Chemistry Letters 2002, 12, (16), 2097-2100).

Less drastic methods may be carried out starting from an aromatic ring substituted with a halogen or an amino group. Then starting from 3-bromopyridine, chlorosulfonation could be achieved in a two steps manner, a first nucleophilic substitution with methylthiolate in excess, to obtain the expected aromatic thiol after acidification, followed by oxidative chlorination of the thiol derivative with chlorine (Maslankiewicz, A.; Marciniec, K.; Pawlowski, M.; Zajdel, P., From haloquinolines and halopyridines to quinoline- and pyridinesulfonyl chlorides and sulfonamides. Heterocycles 2007, 71, (9), 1975-1990) or sodium perchlorate (Zajdel, P.; Marciniec, K.; Maslankiewicz, A.; Grychowska, K.; Satala, G.; Duszynska, B.; Lenda, T.; Siwek, A.; Nowak, G.; Partyka, A.; Wrobel, D.; Jastrzebska-Wiesek, M.; Bojarski, A. J.; Wesolowska, A.; Pawlowski, M., Antidepressant and antipsychotic activity of new quinoline- and isoquinoline-sulfonamide analogs of aripiprazole targeting serotonin 5-HT1A/5-HT2A/5-HT7 and dopamine D-2/D-3 receptors. European Journal of Medicinal Chemistry 2013, 60, 42-50) or oxidation of a corresponding aromatic sulfide with 2,4-dichloro-5,5-dimethylhydantoin (DCDMH)(Pu, Y. M.; Christesen, A.; Ku, Y. Y., A simple and highly effective oxidative chlorination protocol for the preparation of arenesulfonyl chlorides. Tetrahedron Letters 2010, 51, (2), 418-421). Finally, starting from the aromatic amine substrate, which could be obtained by Curtius rearrangement from the corresponding carboxylic acid, the diazotation with $NaNO_2$/HCl followed by chlorosulfonation with $SOCl_2$ in the presence of copper catalyst, could furnish the expected sulfonylchloride derivative (Philip J. Hogan, Brian G. Cox; Aqueous Process Chemistry: The Preparation of Aryl Sulfonyl Chlorides. Org. Process Res. Dev., 2009, 13 (5), pp 875-879).

The present invention also relates to an agrochemical composition, in particular a pesticide composition, comprising at least one compound as defined above, in particular a compound having the formula (I), (II) or (III).

The agrochemical composition of the invention is preferably a pesticide composition chosen from the following compositions: acaricides, insecticides, nematicides, and molluscicides.

The agrochemical composition of the invention may contain further ingredients, which are well-known from the skilled person.

The present invention also relates to the use of a compound as defined above, having the formula (I), (II) or (III), as a pesticide.

According to an embodiment, the present invention relates to the use of a compound of formula (III), as an insecticide.

Preferably, the present invention relates to the use, as an insecticide, of a compound chosen from the following compounds:

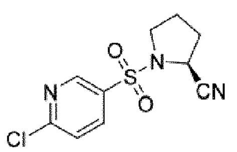 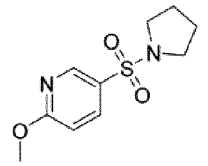 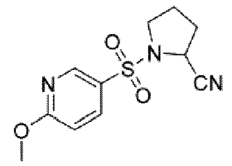 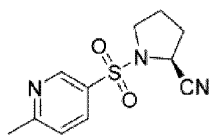

The present invention also relates to a method for pest control in plants or for animal welfare, comprising the application of at least one compound as defined above, to a plant, a plant seed, a plant part, fruit or an animal skin.

According to an embodiment, the present invention relates to a method for pest control in plants or for animal welfare, comprising the application of at least one compound of formula (III), to a plant, a plant seed, a plant part, fruit or an animal skin.

Preferably, the above-mentioned method comprises the application of at least one compound chosen from the following compounds:

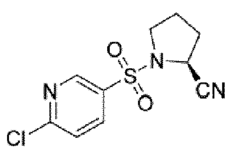 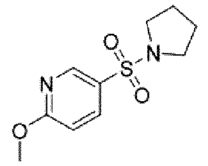 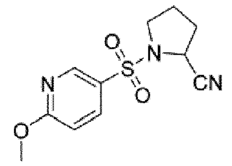 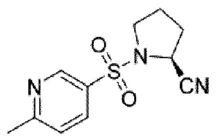

The present invention also relates to a compound as defined above, having the formula (I), (II) or (III), for its use in the prevention of vector-borne diseases.

According to the invention, the vector-borne diseases are human illnesses caused by parasites, viruses and bacteria that are transmitted by mosquitoes, sandflies, triatomine bugs, blackflies, ticks, tsetse flies, mites, snails and lice.
As vector-borne diseases, the followings may be mentioned: malaria, dengue, schistosomiasis, human African trypanosomiasis, leishmaniasis, Chagas disease, yellow fever, Japanese encephalitis and onchocerciasis.

## FIGURES

**Figure 1:** Effect of compound **3i** on synaptic cholinergic transmission.
**Figure 2:** Effect of compound **3ii** on synaptic cholinergic transmission.
**Figure 3:** Effect of compound **3iii** on synaptic cholinergic transmission.
**Figure 4:** Effect of compound **3iv** on synaptic cholinergic transmission.

## MOLECULAR MODELING

***Material and Methods***

### VIRTUAL SCREENING

On the basis of the higher affinity of THI to insect nAChRs compared to the other neonicotinoids (Talley, T. T.; Harel, M.; Hibbs, R. E.; Radic, Z.; Tomizawa, M.; Casida, J. E.; Taylor, P., Atomic interactions of neonicotinoid agonists with AChBP: Molecular recognition of the distinctive electronegative pharmacophore. Proceedings of the National Academy of Sciences of the United States of America 2008, 105, (21), 7606-7611), the THI chemical structure was used as reference for a virtual screening, based on shape similarity, of the Zinc database containing approximately 6 million of compounds (lead-like subset)(Irwin, J. J.; Shoichet, B. K., ZINC - A free database of commercially available compounds for virtual screening. Journal of Chemical Information and Modeling 2005, 45, (1), 177-182). For this step, the Openeye software was employed using ROCS, the Openeye shape screening module (Hawkins, P.C.D.; Skillman, A.G. and Nicholls, A., Comparison of Shape-Matching and Docking as Virtual Screening Tools, Journal of Medicinal Chemistry, Vol. 50, pp. 74-82, 2007; ROCS 3.2.2.2: OpenEye Scientific Software, Santa Fe, NM.).
The sixteen compounds showing the best similarity indices (Shape Tanimoto index, maximum values of 1.0) with THI (values greater than 0.6) have then been docked in cockroach and honeybee nAChRs with the Glide program of the 2014.1 Schrödinger suite (Friesner, R. A.; Banks, J. L.; Murphy, R. B.; Halgren, T. A.; Klicic, J. J.; Mainz, D. T.; Repasky, M. P.; Knoll, E. H.; Shelley, M.; Perry, J. K., Glide: a new approach for rapid, accurate docking and scoring. 1. Method and assessment of docking accuracy. Journal of medicinal chemistry 2004, 47, (7), 1739-1749). More details concerning the homology models used are given elsewhere (Selvam, B.; Graton, J.; Laurent, A. D.; Alamiddine, Z.; Mathe-Allainmat, M.; Lebreton, J.; Coqueret, O.; Olivier, C.; Thany, S. H.; Le Questel, J.-Y., Imidacloprid and thiacloprid neonicotinoids bind more favourably to cockroach than to honeybee alpha 6 nicotinic acetylcholine receptor: Insights from computational studies. Journal of Molecular Graphics & Modelling 2015, 55, 1-12). The ligands were prioritized according to (i) their protonation state (ii) the docking scores (iii) the Glide interactions energies. In any case, the compounds were docked in their neutral state in order to avoid cross reactivity with other nAChRs, especially with human nAChRs whose agonist carries a net positive charge (ammonium group). This approach finally led to seven compounds, among which molecules of series 1 (see Scheme 1).
The virtual screening procedure described above, using the lead-like subset of the ZINC database, led to 16 compounds with a Tanimoto index superior to 0.6. Each of these compounds was carefully examined before the docking stage. The chemical structure of most of these compounds (except two molecules) has been modified to fulfill several criteria. First, if the compounds exist under several protonation states, only the neutral form was retained for further analysis. Furthermore, when the ZINC compounds bear aromatic rings, heteroatoms have been introduced in relevant positions to increase the potential of specific molecular interactions (for example a benzene substituent was changed into a pyridine). Lastly, aliphatic groups (Me, *i*-Pr) carried by heterocyclic rings have generally been removed.

### DOCKING

The structures of the 16 compounds have been converted to 3D using the LigPrep v3.0 (Schrödinger Release 2014-1: LigPrep, version 2.9, Schrödinger, LLC, New York, NY, 2014) module of the Schrödinger suite 2014-1 (Schrödinger Release 2014-1: Schrödinger, LLC, New York, NY, 2014). The 3D ligand molecules were then subjected to the confgen (Schrödinger Release 2014-1: ConfGen, version 2.7, Schrödinger, LLC, New York, NY, 2014) program to retrieve the lowest energy conformer for docking. The docking was performed using the Glide v6.3 (Friesner, R. A.;

Banks, J. L.; Murphy, R. B.; Halgren, T. A.; Klicic, J. J.; Mainz, D. T.; Repasky, M. P.; Knoll, E. H.; Shelley, M.; Perry, J. K., Glide: a new approach for rapid, accurate docking and scoring. 1. Method and assessment of docking accuracy. Journal of medicinal chemistry 2004, 47, (7), 1739-1749) program of the Schrödinger suite 2014-1 (Schrödinger Release 2014-1: Schrödinger, LLC, New York, NY, 2014). The residues around 6 Å of the ligand were defined as the active site and were selected for the receptor grid generation. The extra-precision (XP)( Friesner, R.A., Murphy, R.B., Repasky, M.P., Frye, L.L., Greenwood, J.R., Halgren, T.A., Sanschagrin, P. C., Mainz, D. T. Extra precision glide: docking and scoring incorporating a model of hydrophobic enclosure for protein-ligand complexes. Journal of medicinal chemistry. 2006, 49, 6177-96) mode of the docking algorithm was employed to dock the various ligands. The docking results were validated by comparing the predicted ligand binding modes to the crystallographic *Ac*-AChBP-neonicotinoid structures available (in the present case, the 3C84 entry, which corresponds to the complex between thiacloprid and *Ac*-AChBP).

***Results***

Table 1 shows the docking scores and Glide energies obtained following the docking of the 16 compounds considered in the binding sites of α6 cockroach and honeybee homomeric nAChRs. Since agonists of human nAChRs are known to carry a positive charge (often an ammonium group), as recalled above, only compounds coming out from the virtual screening and that cannot be easily protonated at physiological p*H* were considered, to avoid any cross reactivity.

**Table 1.** Docking scores and Glide energies (kcal/mol) computed following the docking of the top 16 compounds coming out from the virtual screening study on α6 cockroach and honeybee nAChRs.

| | **α6 cockrroach** | | **α6 honeybee** | |
|---|---|---|---|---|
| **Number** | **DS[a]** | **GE[b]** | **DS[a]** | **GE[b]** |
| **1** | -9.7 | -58.3 | -7.6 | -42.1 |
| **2** | -8.1 | -51.7 | -8.2 | -63.2 |
| **3** | -6.1 | -45.6 | -5.1 | -28.1 |
| **4** | -9.1 | -58.2 | -6.7 | -32.7 |
| **5** | -9.7 | -58.3 | -8.2 | -40.0 |
| **6** | -8.2 | -51.9 | -9.2 | -51.6 |
| **7** | -7.1 | -40.8 | -7.7 | -48.9 |
| **8** | -8.4 | -42.9 | -8.3 | -33.9 |
| **9** | -6.0 | -23.4 | -6.1 | -37.2 |
| **10** | -7.1 | -40.7 | -3.7 | -34.3 |
| **11** | -6.4 | -19.9 | -3.8 | -17.7 |
| **12** | -10.2 | -56.5 | -10.8 | -47.8 |
| **13** | -8.9 | -56.7 | -9.9 | -51.1 |
| **14** | -6.2 | -39.9 | -6.1 | -43.7 |
| **15** | -9.3 | -43.2 | -9.5 | -44.1 |
| **16** | -9.9 | -59.1 | c | c |

[a] DS: Docking Score (see methodology section) [b] GE: Glide energy (kcal/mol, see methodology section) [c] The docking of this compound was not possible in honeybee α6 nAChR.

Among the 16 compounds, 7 appear promising (**1**, **3**, **4-5**, **10**, **11**, **16**) from these results, since their docking parameters (docking scores and Glide energies) are significantly more favorable for α6 cockroach nAChRs compared to α6 honey bee nAChRs. Indeed, for **2**, **6**, **8-9** and **14-15**, one, or both of the corresponding parameters have for both insect species very similar values, no selectivity coming out from the results. For one compound (**7**), both values are on the contrary more favorable for α6 honey bee nAChRs, suggesting a possible selectivity for these receptors. Finally, for two compounds (**12**, **13**), no clear conclusions can be drawn from the results since the trends suggested by the docking scores and the Glide energies are opposite.

The interactions of compound **3** in the binding site of α6 cockroach and honeybee nAChRs have been studied. A first important interaction corresponds to a hydrogen-bond between the pyridine nitrogen of the ligand and main chain CO and NH groups of the receptor through a water molecule in both insect species. This feature agrees with the role played by a water molecule that appears conserved in several cocrystallized ligand-nAChR model complexes (Jeschke, P.; Nauen, R.; Beck, M. E., Nicotinic Acetylcholine Receptor Agonists: A Milestone for Modern Crop Protection. Angewandte Chemie-International Edition 2013, 52, (36), 9464-9485; Blum, A. P.; Lester, H. A.; Dougherty, D. A., Nicotinic pharmacophore: The pyridine N of nicotine and carbonyl of acetylcholine hydrogen bond across a subunit interface to a backbone NH. Proceedings of the National Academy of Sciences of the United States of America 2010, 107, (30), 13206-13211; Xiao, Y.; Hammond, P. S.; Mazurov, A. A.; Yohannes, D., Multiple Interaction Regions in the Orthosteric Ligand Binding Domain of the alpha 7 Neuronal Nicotinic Acetylcholine Receptor. Journal of Chemical Information and Modeling 2012, 52, (11), 3064-3073; and Amiri, S.; Sansom, M. S. P.; Biggin, P. C., Molecular dynamics studies of AChBP with nicotine and carbamylcholine: the role of water in the binding pocket. Protein Engineering Design & Selection 2007, 20, (7), 353-359) and has been suggested to be incorporated in the pharmacophore for the design of new ligands.

It has also been shown then that in both cases, Trp residues (Trp 175 and 200 for α6 cockroach and honeybee nAChRs, respectively) have a pivotal contribution in the binding of the ligand, more precisely with the five membered saturated ring. These trends are in line with the prominence of this residue pointed out by experimental studies (Blum, A. P.; Lester, H. A.; Dougherty, D. A., Nicotinic pharmacophore: The pyridine N of nicotine and carbonyl of acetylcholine hydrogen bond across a subunit interface to a backbone NH. Proceedings of the National Academy of Sciences of the United States of America 2010, 107, (30), 13206-13211; Blum, A. P.; Gleitsman, K. R.; Lester, H. A.; Dougherty, D. A., Evidence for an Extended Hydrogen Bond Network in the Binding Site of the Nicotinic Receptor Role of the Vicinal Disulfide of the Alpha 1 Subunit. Journal of Biological Chemistry 2011, 286, (37), 32251-32258; Puskar, N. L.; Xiu, X.; Lester, H. A.; Dougherty, D. A., Two Neuronal Nicotinic Acetylcholine Receptors, alpha 4 beta 4 and alpha 7, Show Differential Agonist Binding Modes. Journal of Biological Chemistry 2011, 286, (16), 14618-14627; Xiu, X.; Puskar, N. L.; Shanata, J. A. P.; Lester, H. A.; Dougherty, D. A., Nicotine binding to brain receptors requires a strong cation-π interaction. Nature (London, U. K.) Nature (London, United Kingdom) 2009, 458, (7237), 534-537; Sine, S. M., End-plate acetylcholine receptor: structure, mechanism, pharmacology, and disease. Physiological Reviews 2012, 92, (3), 1189-1234; Rucktooa, P.; Smit, A. B.; Sixma, T. K., Insight in nAChR subtype selectivity from AChBP crystal structures. Biochemical Pharmacology 2009, 78, (7, Sp. Iss. SI), 777-787; and Celie, P. H. N.; Van Rossum-Fikkert, S. E.; Van Dijk, W. J.; Brejc, K.; Smit, A. B.; Sixma, T. K., Nicotine and carbamylcholine binding to nicotinic acetylcholine receptors as studied in AChBP crystal structures. Neuron 2004, 41, (6), 907-914) and rationalized by computational investigations (Selvam, B.; Graton, J.; Laurent, A. D.; Alamiddine, Z.; Mathe-Allainmat, M.; Lebreton, J.; Coqueret, O.; Olivier, C.; Thany, S. H.; Le Questel, J.-Y., Imidacloprid and thiacloprid neonicotinoids bind more favourably to cockroach than to honeybee alpha 6 nicotinic acetylcholine receptor: Insights from computational studies. Journal of Molecular Graphics & Modelling 2015, 55, 1-12; Alamiddine, Z.; Selvam, B.; Ceron-Carrasco, J. P.; Mathe-Allainmat, M.; Lebreton, J.; Thany, S. H.; Laurent, A. D.; Graton, J.; Le Questel, J.-Y., Molecular recognition of thiaclopride by Aplysia californica AChBP: new insights from a computational investigation. J. Comput.-Aided Mol. Des. 2015, 29, (Copyright (C) 2016 American Chemical Society (ACS). All Rights Reserved.), 1151-1167; Atkinson, A.; Graton, J.; Le, Q. J.-Y., Insights into a highly conserved network of hydrogen bonds in the agonist binding site of nicotinic acetylcholine receptors: a structural and theoretical study. Proteins 2014, 82, (Copyright (C) 2016 U.S. National Library of Medicine.), 2303-17; and Ceron-Carrasco, J. P.; Jacquemin, D.; Graton, J.; Thany, S.; Le Questel, J.-Y., New Insights on the Molecular Recognition of Imidacloprid with Aplysia californica AChBP: A Computational Study. Journal of Physical Chemistry B 2013, 117, (Copyright (C) 2016 American Chemical Society (ACS). All Rights Reserved.), 3944-3953).

Lastly, it is worth noticing that the key cysteine residues are in the two binding sites in close vicinity with the ligand, the sulfur atom of one of those (Cys219 and 249 in α6 cockroach and honeybee nAChRs, respectively) being in close contact with the oxygen atom of the sulfonamide group. This feature highlights the possible potential of interaction of this functional fragment, found in recent nAChRs modulators acting as insecticides, in particular in sulfoxaflor, designed by Dow Agrosciences (Sparks, T. C.; Watson, G. B.; Loso, M. R.; Geng, C.; Babcock, J. M.; Thomas, J. D., Sulfoxaflor and the sulfoximine insecticides: Chemistry, mode of action and basis for efficacy on resistant insects. Pesticide Biochemistry and Physiology 2013, 107, (1), 1-7; Wang, N. X.; Watson, G. B.; Loso, M. R.; Sparks, T. C., Molecular modeling of sulfoxaflor and neonicotinoid binding in insect nicotinic acetylcholine receptors: impact of the Myzus β1 R81T mutation. Pest Manage. Sci. 2016, 72, , 1467-1474; and Cutler, P.; Slater, R.; Edmunds, A. J. F.; Maienfisch, P.; Hall, R. G.; Earley, F. G. P.; Pitterna, T.; Pal, S.; Paul, V.-L.; Goodchild, J.; Blacker, M.; Hagmann, L.; Crossthwaite, A. J., Investigating the mode of action of sulfoxaflor: a fourth-generation neonicotinoid. Pest Management Science 2013, 69, (5), 607-619).

From this analysis, no clear difference of interactions appears therefore for **3** for cockroach and honeybee α6 nAChRs binding sites. In fact, a further examination of the interaction energies rationalizes the better affinity for α6 cockroach nAChR. Indeed, Table 2 shows that for the main aminoacid residues involved in the binding and discussed above, the stabilization is significantly greater for cockroach α6 nAChRs. The present molecular modeling results, validated by their good agreement with known (experimental) features for the interaction of nAChRs modulators and their target, are therefore promising for the insecticide activity of **3** and its selectivity for pests.

**Table 2**. Interaction energies (kcal/mol) computed by the Glide program for the main components of the a6 cockroach and honeybee nAChRs binding sites. Docking scores and Glide energies (kcal/mol) are reminded for clarity.

| | $\alpha$6 cock (roach nAC ChR | | | | | | |
|---|---|---|---|---|---|---|---|
| **3** 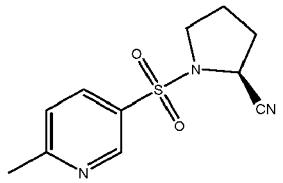 | **Trp81** | **Val145** | **Trp175** | **Tyr217** | **Cys219** | **DS** | **GE** |
| | -2.6 | -5.1 | -5.5 | -4.8 | -2.5 | -6.1 | -45.6 |
| | $\alpha$6 honeybee nAChR | | | | | | |
| | **Trp106** | **Val170** | **Trp200** | **Tyr242** | **Cys245** | **DS** | **GE** |
| | >0 | -2.3 | -4.5 | -2.9 | -2.0 | -5.1 | -23.6 |

## PREPARATION OF COMPOUNDS OF FORMULA (I)

The compounds of the invention having the formula (I) displayed a sulfonamide function and could be simply prepared from nucleophilic substitution of a sulfonyl chloride precursor **A** with the selected pyrrolidine **B**, for example according to the below scheme (Scheme 1):

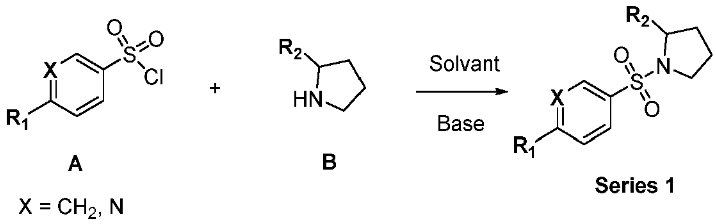


**A** **B** **Series 1**

X = $CH_2$, N

*Scheme 1*

Access to such (hetero)aromatic sulfonyl chloride compounds (**A**) could be achieved by conventional procedures such as the method illustrated on Scheme 2.

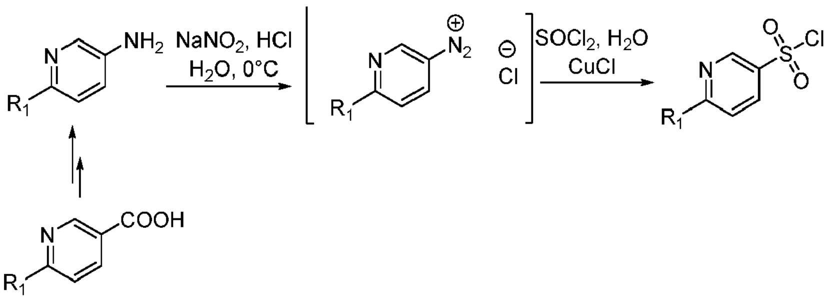


*Scheme 2*

The compounds were prepared by applying the one-pot two steps Sandmeyer-sulfonation approach (Scheme 2) starting from aniline or 3-amino pyridine precursors to prepare the selected chlorosulfonyl reagents. These were obtained with good yield excepted for compound **1d** which failed to precipitate in this aqueous media (Scheme 3).

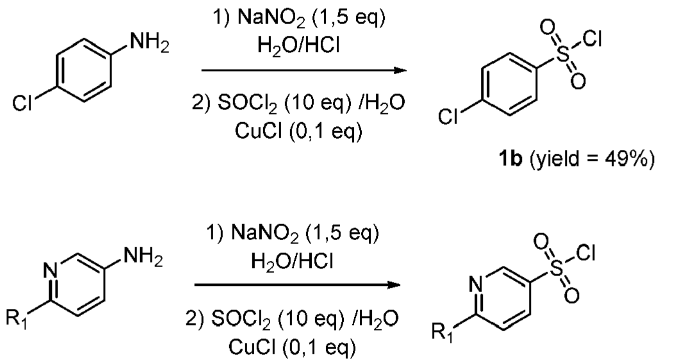


**1b** (yield = 49%)

**1c** ($R_1$ = OMe, yield = 60%)
**1d** ($R_1$ = Cl, yield = 63%)
**1e** ($R_1$ = Me, yield = 5%)

*Scheme 3: Synthesis of the selected aryl sulfonyl chloride reagents*

To access to the racemic 2-cyano pyrrolidine **B** ($R_2$ = CN)(corresponding to a compound of formula (V) as defined above with R=CN), two synthetic ways were proposed in the literature, either starting from 4,4-diethoxy-*N*,*N*-dimethyl-1-butanamine, by a Strecker reaction (Bahde, R. J.; Rychnovsky, S. D., Cyclization via Carbolithiation of α-Amino Alkyllithium Reagents. Organic Letters 2008, 10, (18), 4017-4020)(Scheme 4, method a) or starting from a triazine intermediate followed by a cyanation reaction with HCN (De Kimpe, N. G.; Stevens, C. V.; Keppens, M. A., Synthesis of 2-acetyl-1-pyrroline, the principal rice flavor component. Journal of Agricultural and Food Chemistry 1993, 41, (9), 1458-1461) or TMSCN (Liu, X.-W.; Le, T. N.; Lu, Y.; Xiao, Y.; Ma, J.; Li, X., An efficient synthesis of chiral phosphinyl oxide pyrrolidines and their application to asymmetric direct aldol reactions. Organic & Biomolecular Chemistry 2008, 6, (21), 3997-4003; and Köhler, V.; Bailey, K. R.; Znabet, A.; Raftery, J.; Helliwell, M.; Turner, N. J., Enantioselective Biocatalytic Oxidative Desymmetrization of Substituted Pyrrolidines. Angewandte Chemie International Edition 2010, 49, (12), 2182-2184)(Scheme 4, method b).

Applying method b (Scheme 4) racemic pyrrolidine **2b** was prepared in 34% overall yield on two steps. The synthesis of the (S) enantiomer of 2-cyano pyrrolidine was also described in the literature starting from Boc-proline (Ji, X.; Su, M.; Wang, J.; Deng, G.; Deng, S.; Li, Z.; Tang, C.; Li, J.; Li, J.; Zhao, L.; Jiang, H.; Liu, H., Design, synthesis and biological evaluation of hetero-aromatic moieties substituted pyrrole-2-carbonitrile derivatives as dipeptidyl peptidase IV inhibitors. European Journal of Medicinal Chemistry 2014, 75, 111-122).

**Method a**

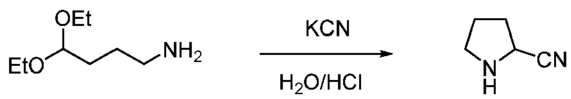


**2b**

**Method b**

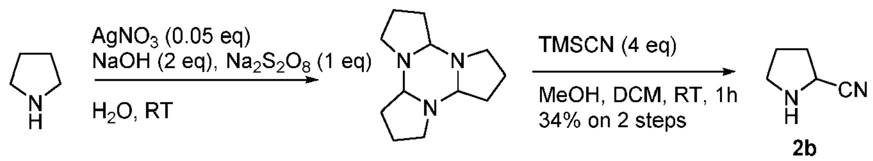


**2b**

*Scheme 4: Synthesis of the substituted pyrrolidine 2b*

To synthesize the sulfonamides of the invention, the following conditions were preferred and a series of compounds **3** (**3aa- 3ec**) were obtained starting from commercial aniline **1a** (Scheme 5) or synthesized (hetero)aromatic amines **1b-e** (Schemes 6-9).

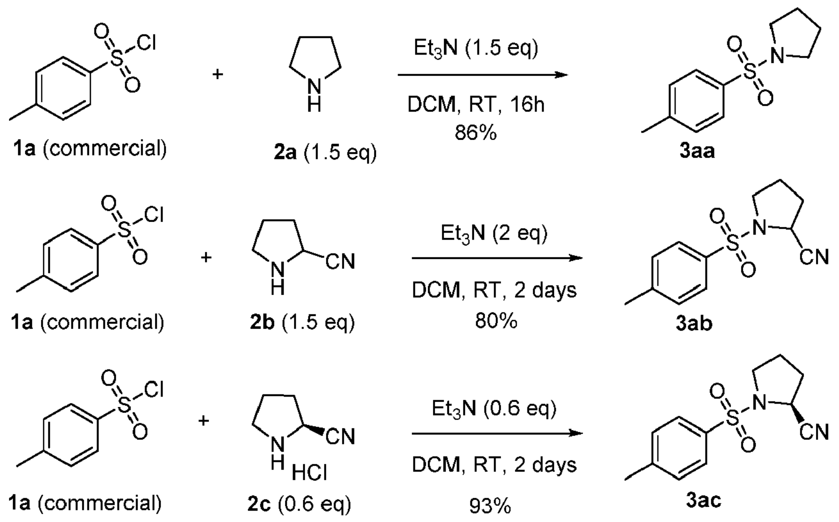


**1a** (commercial) + **2a** (1.5 eq) → **3aa**

**1a** (commercial) + **2b** (1.5 eq) → **3ab**

**1a** (commercial) + **2c** (0.6 eq) → **3ac**

*Scheme 5*

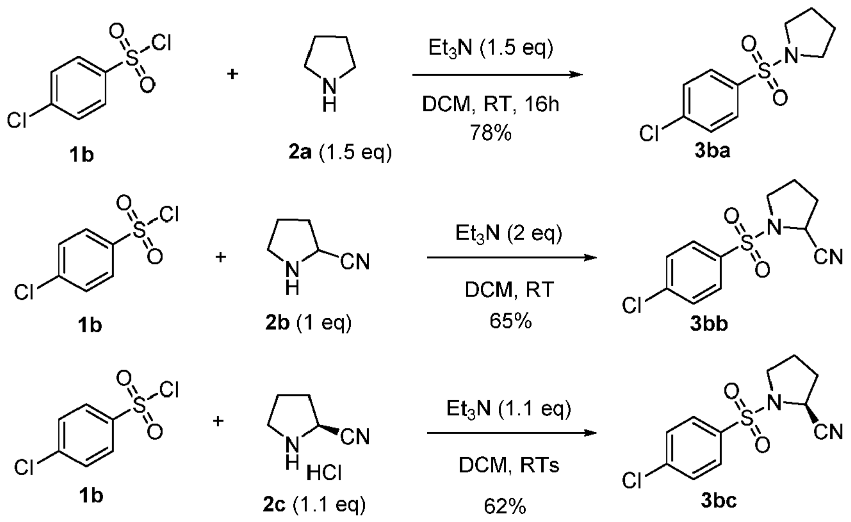


**1b** + **2a** (1.5 eq) → **3ba**

**1b** + **2b** (1 eq) → **3bb**

**1b** + **2c** (1.1 eq) → **3bc**

*Scheme 6*

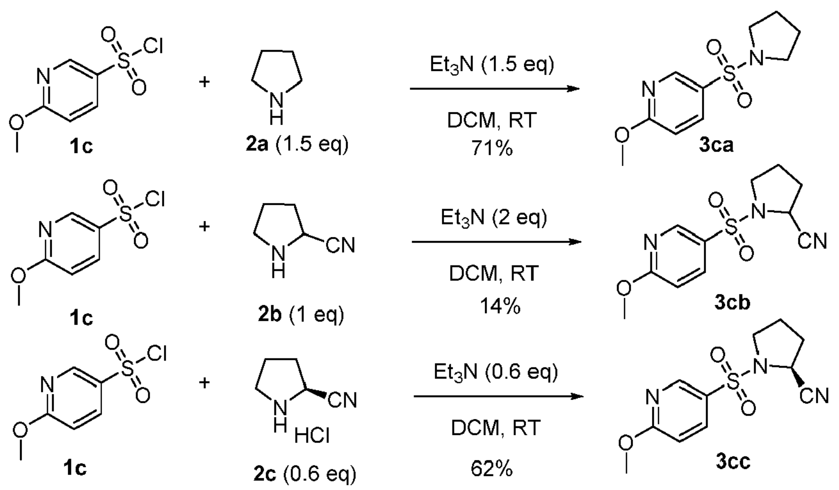


**1c** + **2a** (1.5 eq) → **3ca**

**1c** + **2b** (1 eq) → **3cb**

**1c** + **2c** (0.6 eq) → **3cc**

*Scheme 7*

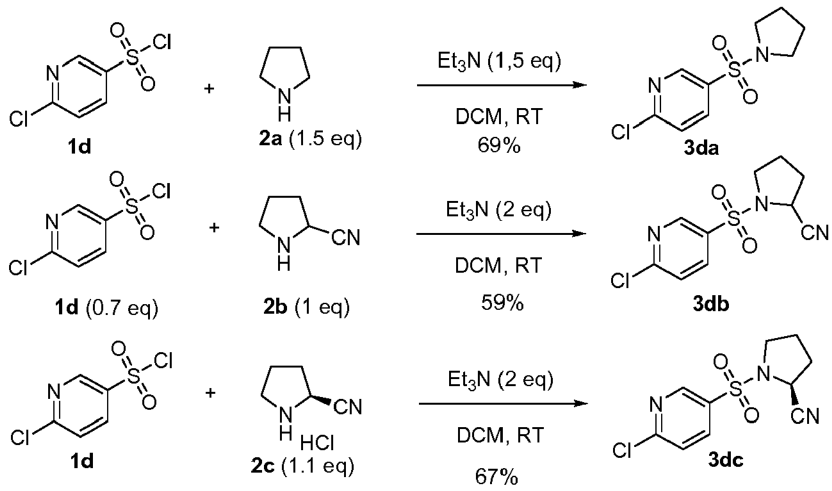


*Scheme 8*

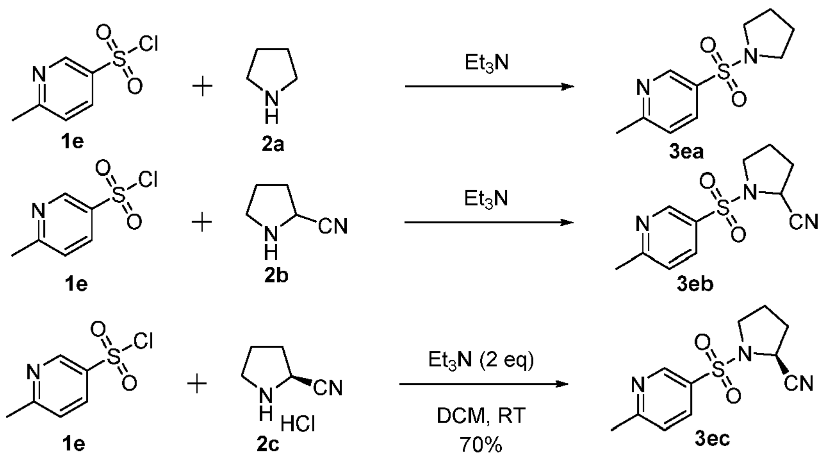


*Scheme 9*

**Chemistry.** *General.* All solvents used were reagent grade and TLC was performed on silica-covered aluminum sheets (Kieselgel 60F254, MERCK). Eluted TLC was revealed using UV radiation ($\lambda$ = 254 nm), or molybdate solution. Flash column chromatography was performed on silica gel 60 ACC 40-63 $\mu$m (SDS-CarloErba). NMR spectra were recorded on a BRUKER AC300 (300 MHz for $^1$H and 75 MHz for $^{13}$C) or on a BRUKER 400 (400 MHz for $^1$H and 100 MHz for $^{13}$C) at room temperature, on samples dissolved in an appropriate deuterated solvent. References of tetramethylsilane (TMS) for 1H and deuterated solvent signal for $^{13}$C were used.

Chemical displacement values ($\delta$) are expressed in parts per million (ppm), and coupling constants (*J*) in Hertz (Hz). Low-resolution mass spectra (MS in Da unit) were recorded in the CEISAM laboratory on a Thermo-Finnigan DSQII quadripolar at 70 eV (CI with $NH_3$ gas) or on a Waters Xevo G2-XS QTOF. High-resolution mass spectra (HRMS) were recorded on ESI or MALDI with Q-Tof analyzers within a tolerance of 5 ppm of the theoretically calculated value and measurements are given in Da. Infrared spectra were recorded on a IRTF spectrophotometer (Bruker Vertex 70). Optical rotation data were obtained on a polarimeter, in a 100 mm cell, under Na lamp radiation at 20 °C.

**Example 1:** *Compound **3aa:** preparation of 1-tosylpyrrolidine*

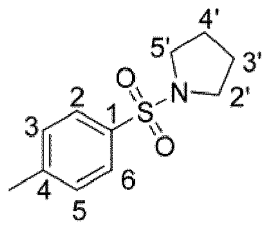

**Formula:** $C_{11}H_{15}NO_2S$
**Molecular weight:** 225.31 g.mol$^{-1}$

To a solution of 4-methylbenzenesulfonyl chloride (217 mg, 1.14 mmol, 1 eq) in methylene chloride (10 mL) were added pyrrolidine (121.4 mg, 0.14 mL, 1.71 mmol, 1.5 eq) and triethylamine (172.8 mg, 0.24 mL, 1.71 mmol, 1.5 eq). The mixture was stirred during 16 h, hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 86% yield.
**mp:** 129°C
**$^1$H NMR (300 MHz, $CDCl_3$):** δ 7.71 (d, 2H, 8.1 Hz, H2 and H6); 7.31 (d, 2H, 8.1 Hz, $H_3$ and $H_5$); 3.25-3.20 (m, 4H, $H_{2',\alpha}$, $H_{2',\beta}$, $H_{5',\alpha}$ and $H_{5',\beta}$); 2.43 (s, 3H, $CH_3$); 1.76-1.72 (m, 4H, $H_{3',\alpha}$, $H_{3',\beta}$, $H_{4',\alpha}$ and $H_{4',\beta}$).
**$^{13}$C NMR (75 MHz, $CDCl_3$):** δ 143.3 ($C_4$); 133.9 ($C_1$); 129.6 (2C, $C_3$ and $C_5$); 127.6 (2C, $C_2$ and $C_6$); 47.9 (2C, $C_{2'}$ and $C_{5'}$); 25.2 (2C, $C_{3'}$ and $C_{4'}$); 21.5 ($CH_3$).
MS: ESI+: $[M+H]^+$ = 226.1; $[M+Na]^+$ = 248.0.
HRMS (ESI+) calculated for $C_{11}H_{15}NNaO_2S$ [M+Na$^+$] m/z = 248.0721 found 248.0726.
IR ATR: ν (cm$^{-1}$) 3092.45-3035.04 (=C-H); 2975.45-2866.37 (-C-H); 1330.87 ($SO_2$,as); 1105.81 ($SO_2$,s).

**Example 2:** *Compound **3ab:** preparation of 1-tosylpyrrolidine-2-carbonitrile*

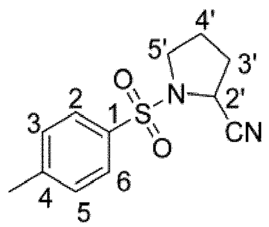

**Formula:** $C_{12}H_{14}N_2O_2S$
**Molecular weight:** 250.32 g.mol$^{-1}$

To a solution of pyrrolidine-2-carbonitrile (113.5 mg, 1.18 mmol, 1.1 eq) in methylene chloride (10 mL) were added triethylamine (217.2 mg, 0.3 mL, 2.15 mmol, 2 eq) and 4-methylbenzenesulfonyl chloride (204.6 mg, 1.07 mmol, 1 eq). The mixture was stirred during 2 days then hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 80% yield.
**mp:** 102°C
**$^1$H NMR (400 MHz, $CDCl_3$):** δ 7.78 (d, 2H, 8.4 Hz, $H_3$ and $H_5$); 7.35 (d, 2H, 8.4 Hz, $H_2$ and $H_6$); 4.59 (dd, 1H, 6 Hz and 2.4 Hz, $H_{2'}$); 3.43-3.33 (m, 2H, $H_{5',\alpha}$ and $H_{5',\beta}$); 2.44 (s, 3H, $CH_3$); 2.25-1.95 (m, 4H, $H_{3',\alpha}$, $H_{3',\beta}$, $H_{4',\alpha}$ and $H_{4',\beta}$).
**$^{13}$C NMR (100 MHz, $CDCl_3$):** δ 144.5 ($C_4$); 134.4 ($C_1$); 129.9 (2C, $C_3$ and $C_5$); 127.6 (2C, $C_2$ and $C_6$); 118.0 (CN); 48.6 ($C_{2'}$); 47.4 ($C_{5'}$); 31.9 ($C_{3'}$); 24.6 ($C_{4'}$); 21.6 ($CH_3$).
**MS:** CI+ : $[M+NH_4]^+$ = 267.4; CI- : $[M-H]^-$ = 249.3.
**HRMS:** HRMS (ESI+) calculated for $C1_2H_{15}N_2O_2S$ $[M+H]^+$ m/z = 251.0849 found 251.0847. **HPLC:** IA; Heptane/$CH_2Cl_2$ 8/2; 0.8 mL.min$^{-1}$; 20°C; 230 nm: tr = 28.57 min and 30.67.
**IR ATR:** ν (cm$^{-1}$) 3022.04-3001.45 (=C-H); 2995.15-2874.79 (-C-H); 2247.57 (CN); 1343.78 ($SO_2$,as); 1154.49 ($SO_2$,s).

Example 3: *Compound **3ac**: preparation of (S)-1-tosylpyrrolidine-2-carbonitrile*

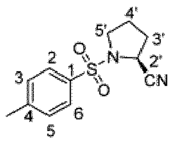

**Formula:** $C_{12}H_{14}N_2O_2S$
**Molecular weight:** 250.32 g-mol$^{-1}$

To a mixture of (S)-pyrrolidine-2-carbonitrile hydrochloride (76.5 mg, 0.88 mmol, 1.1 eq) in methylene chloride (10 mL) was added triethylamine (106 mg, 0.15 mL, 1.05 mmol, 2 eq). The mixture was stirred during 15 min, 4-methylbenzenesulfonyl chloride (100 mg, 0.52 mmol, 1 eq) was added and the stirring was continued for additional 16 h. The mixture was then hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 93% yield.
**mp:** 102°C
**$^1$H NMR (300 MHz, $CDCl_3$):** δ 7.78 (d, 2H, 8.4 Hz, $H_2$ and $H_6$); 7.35 (d, 2H, 8.4 Hz, $H_3$ and $H_5$); 4.59 (dd, 1H, 6 Hz and 2.4 Hz, $H_2$); 3.41-3.36 (m, 2H, $H_{5',\alpha}$ and $H_{5',\beta}$); 2.44 (s, 3H, $CH_3$); 2.23-2.06 (m, 4H, $H_{3',\alpha}$, $H_{3',\beta}$, $H_{4',\alpha}$ and $H_{4',\beta}$).
**$^{13}$C NMR (75 MHz, $CDCl_3$):** δ 144.5 ($C_4$); 134.4 ($C_1$); 129.9 (2C, $C_3$ and $C_5$); 127.6 (2C, $C_2$ and $C_6$); 118.0 (CN); 48.6 ($C_{2'}$); 47.4 ($C_{5'}$); 31.9 ($C_{3'}$); 24.6 ($C_{4'}$); 21.6 ($CH_3$).
**MS:** ESI+: $[M-CN]^+$ = 224.1; $[M+Na]^+$ = 273.0.
**HRMS:** HRMS (ESI+) calculated for $C_{12}H_{14}N_2NaO_2S$ $[M+Na^+]$ m/z = 273.0674 found 273.0681.
**$[\alpha]_D^{20°C}$**: -108.6 (1 g/100 mL) in MeOH.
**HPLC:** IA; Heptane/$CH_2Cl_2$ 8/2; 0.8 mL.min$^{-1}$; 20°C; 230 nm: tr = 30.75 min.
**IR ATR:** v (cm-1) 3053.36-3006.84 (=C-H); 2986.91-2874.48 (-C-H); 2240.83 (CN); 1334.25 ($SO_2$,as); 1109.57 ($SO_2$,s).

Example 4: *Compound **3ba**: preparation of 1-((4-chlorophenyl)sulfonyl) pyrrolidine*

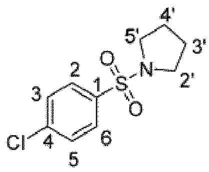

**Formula:** $C_{10}H_{12}ClNO_2S$
**Molecular weight:** 245,72 g.mol$^{-1}$

To a solution of 4-chlorobenzenesulfonyl chloride (235 mg, 1.11 mmol, 1 eq) in methylene chloride (10 mL) was added pyrrolidine (68.5 mg, 0.08 mL, 0.96 mmol, 0.9 eq). The mixture was stirred during 16 h, hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 8/2) to afford the product as a white solid in 78% yield.
**mp:** 106°C
**$^1$H NMR (300 MHz, DMSO-*d6*):** δ 7.82 (d, 2H, 8.4 Hz, $H_2$ and $H_6$); 7.70 (d, 2H, 8.4 Hz, $H_3$ and $H_5$); 3.15-3.11 (m, 4H, $H_{2',\alpha}$, $H_{2',\beta}$, $H_{5',\alpha}$ and $H_{5',\beta}$); 1.67-1.63 (m, 4H, $H_{3',\alpha}$, $H_{3',\beta}$, $H_{4',\alpha}$ and $H_{4',\beta}$).
**$^{13}$C NMR (75 MHz, DMSO-*d6*):** δ 137.9 ($C_4$); 135.1 ($C_1$); 129.5 (2C, $C_3$ and $C_5$); 129.2 (2C, $C_2$ and $C_6$); 47.8 (2C, $C_{2'}$ and $C_{5'}$); 24.7 (2C, $C_{3'}$ and $C_{4'}$).
**$^1$H NMR (300 MHz, $CDCl_3$):** δ 7.76 (mt, 2H, $H_2$ and $H_6$); 7.50 (mt, 2H, $H_3$ and $H_5$); 3.26-3.21 (m, 4H, $H_{2',\alpha}$, $H_{2',\beta}$, $H_{5',\alpha}$ and $H_{5',\beta}$); 1.80-1.75 (m, 4H, $H_{3',\alpha}$, $H_{3',\beta}$, $H_{4',\alpha}$ and $H_{4',\beta}$).
**$^{13}$C NMR (75 MHz, $CDCl_3$):** δ 139.1 ($C_4$); 135.6 ($C_1$); 129.3 (2C, $C_3$ and $C_5$); 128.9 (2C, $C_2$ and $C_6$); 48.0 (2C, $C_{2'}$ and

$C_{5'}$); 25.3 (2C, $C_{3'}$ and $C_{4'}$).
**MS:** ESI+: $[M+H]^+$ = 246.1; $[M+Na]^+$ = 268.1.
**IR ATR:** ν ($cm^{-1}$) 3087.64-3061.44 (=C-H); 2976.50-2877.84 (-C-H); 1336.63 ($SO_2$,as); 1155.51 ($SO_2$,s).

**Example 5**: *Compound **3bb**: preparation of 1-((4-chlorophenyl)sulfonyl) pyrrolidine-2-carbonitrile*

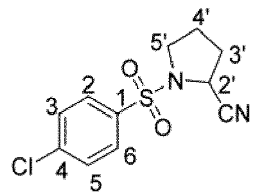

**Formula:** $C_{11}H_{11}ClN_2O_2S$
**Molecular weight:** 270.73 $g.mol^{-1}$

To a solution of pyrrolidine-2-carbonitrile (123.9 mg, 1.45 mmol, 1.1 eq) in methylene chloride (10 mL) were added triethylamine (266 mg, 0.37 mL, 2.63 mmol, 2 eq) and 4-chlorobenzenesulfonyl chloride (277.4 mg, 1.31 mmol, 1 eq). The mixture was stirred during 2 days then hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 7/3) to afford the product as a white solid in 65% yield.
**mp:** 115°C
**$^1$H NMR (400 MHz, $CDCl_3$):** δ 7.85 (mt, 2H, 8.7 Hz, H2 and H6); 7.54 (mt, 2H, 8.7 Hz, $H_3$ and $H_5$); 4.64 (dd, 1H, 6.6 Hz and 3 Hz, $H_{2'}$); 3.44 (mt, 1H, $H_{5',\alpha}$); 3.33 (mt, 1H, $H_{5',\beta}$); 2.28-2.07 (m, 4H, $H_{3',\alpha}$, $H_{3',\beta}$, $H_{4',\alpha}$ and $H_{4',\beta}$).
**$^{13}$C NMR (100 MHz, $CDCl_3$):** δ 140.1 ($C_4$); 136.1 ($C_1$); 129.6 (2C, $C_3$ and $C_5$); 129.0 (2C, $C_2$ and $C_6$); 117.7 (CN); 48.6 ($C_{2'}$); 47.4 ($C_{5'}$); 31.9 ($C_{3'}$); 24.7 ($C_{4'}$).
**MS:** $CI^+$: $[M+NH4^+]^+$ = 287.9.
**HRMS** (ESI+) calculated for $C_{11}H_{11}ClNaN_2O_2S$ $[M+Na^+]$ m/z = 293.0122 found 293.0128.
**HPLC:** IA; Heptane/$CH_2Cl_2$ 75/25; 1 $mL.min^{-1}$; 20°C; 236 nm: tr = 14.36 min and 15.72 min.
**IR ATR:** ν ($cm^{-1}$) 3090.19-3062.53 (=C-H); 2993.31-2870.87 (-C-H); 2249.61 (CN); 1347.37 ($SO_2$,as); 1158.00 ($SO_2$,s).

**Example 6**: *Compound **3bc**: preparation of (S)-1-((4-chlorophenyl)sulfonyl) pyrrolidine-2-carbonitrile*

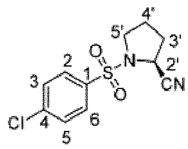

**Formula:** $C_{11}H_{11}ClN_2O_2S$
**Molecular weight:** 270.73 $g.mol^{-1}$

To a mixture of (S)-pyrrolidine-2-carbonitrile hydrochloride (104 mg, 0.78 mmol, 1.1 eq) in methylene chloride (10 mL) was added triethylamine (114 mg, 0.2 mL, 1.42 mmol, 2 eq). The mixture was stirred during 15 min, 4-chlorobenzenesulfonyl chloride (150 mg, 0.71 mmol, 1 eq) was added and the stirring was continued for additional 16 h. The mixture was then hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 62% yield.
**mp:** 115°C
**$^1$H NMR (300 MHz, $CDCl_3$):** δ 7.85 (mt, 2H, 8.7 Hz, $H_2$ and $H_6$); 7.53 (mt, 2H, 8.7 Hz, $H_3$ and $H_5$); 4.64 (dd, 1H, 6.6 Hz and 3 Hz, $H_{2'}$); 3.48-3.31 (m, 2H, $H_{5',\alpha}$ and $H_{5',\beta}$); 2.28-2.07 (m, 4H, $H_{3',\alpha}$, $H_{3',\beta}$, $H_{4',\alpha}$ and $H_{4',\beta}$).

**$^{13}C$ NMR (75 MHz, $CDCl_3$):** δ 140.1 ($C_4$); 136.1 ($C_1$); 129.7 (2C, $C_3$ and $C_5$); 129.0 (2C, $C_2$ and $C_6$); 117.7 (CN); 48.6 ($C_{2'}$); 47.4 ($C_{5'}$); 31.9 ($C_{3'}$); 24.7 ($C_{4'}$).
**MS:** $CI^+$: $[M+NH_4]^+$ = 287.9; ESI+: $[M+Na]^+$ = 293.0.
**HRMS:** (ESI+) calculated for $C_{11}H_{11}ClN_2NaO_2S$ [M+Na+] m/z = 293.0122 found 293.0121.
**$[\alpha]_D^{20°C}$:** -98.8 (1 g/100 mL) in MeOH.
**HPLC:** IA; Heptane/$CH_2Cl_2$ 75/25; 1 mL.min$^{-1}$; 20°C; 236 nm: tr = 14.56 min.
**IR ATR:** ν (cm$^{-1}$) 3093.06-3011.25 (=C-H); 2988.63-2878.83 (-C-H); 2242.49 (CN); 1336.06 ($SO_2$,as); 1155.89 ($SO_2$,s).

Example 7: *Compound **3ca:** preparation of 2-methoxy-5-(pyrrolidin-1-ylsulfonyl)pyridine*

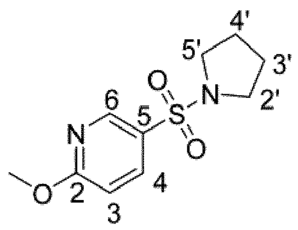

**Formula:** $C_{10}H_{14}N_2O_3S$
**Molecular weight:** 242.29 g.mol$^{-1}$

To a solution of 6-methoxypyridine-3-sulfonyl chloride (180 mg, 0.87 mmol, 1 eq) in methylene chloride (10 mL) were added pyrrolidine (92.5 mg, 0.11 mL, 1.30 mmol, 1.5 eq) and triethylamine (131.6 mg, 0.18 mL, 1.30 mmol, 1.5 eq). The mixture was stirred during 16 h, hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 71% yield.
**mp:** 131°C
**$^1H$ NMR (300 MHz, $CDCl_3$):** δ 8.63 (d, 1H, 2.1 Hz, $H_6$); 7.94 (dd, 1H, 8.7 Hz and 2.1 Hz, $H_4$); 6.83 (d, 1H, 8.7 Hz, $H_3$); 4.00 (s, 3H, $OCH_3$); 3.27-3.22 (m, 4H, $H_{2',\alpha}$, $H_{2',\beta}$, $H_{5',\alpha}$ and $H_{5',\beta}$); 1.81-1.77 (m, 4H, $H_{3',\alpha}$, $H_{3',\beta}$, $H_{4',\alpha}$ and $H_{4',\beta}$).
**$^{13}C$ NMR (75 MHz, $CDCl_3$):** δ 166.4 ($C_2$); 147.4 ($C_6$); 137.6 ($C_4$); 126.5 ($C_5$); 111.3 ($C_3$); 54.2 ($OCH_3$); 47.9 (2C, $C_{2'}$ and $C_{5'}$); 25.3 (2C, $C_{3'}$ and $C_{4'}$).
**MS:** ESI+: $[M+H]^+$ = 243.1; $[M+Na]^+$ = 265.0.
**HRMS** (ESI+) calculated for $C_{10}H_{14}N_2NaO_3S$ [M+Na$^+$] m/z = 265.0623 found 265.0630.
**IR ATR:** ν (cm$^{-1}$) 3096.50-3009.84 (=C-H); 2987.90-2848.51 (-C-H); 1331.32 ($SO_2$,as); 1134.32 ($SO_2$,s).

Example 8: *Compound **3cb**: preparation of 1-((6-methoxypyridin-3-yl)sulfonyl)pyrrolidine-2-carbonitrile*

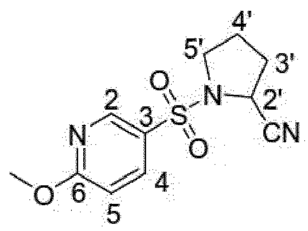

**Formula:** $C_{11}H_{13}N_3O_3S$
**Molecular weight:** 267.30 g.mol$^{-1}$

To a solution of pyrrolidine-2-carbonitrile (134 mg, 1.39 mmol, 1.1 eq) in methylene chloride (10 mL) were added triethylamine (256.5 mg, 0.35 mL, 2.53 mmol, 1.5 eq) and 6- methoxypyridine-3-sulfonyl chloride (263 mg, 1.27 mmol, 1 eq). The mixture was stirred during 2 days then hydrolyzed with an aqueous solution of HCl (2M) and extracted

with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 14% yield.

**mp:** 109°C

**RMN $^1$H NMR (400 MHz, $CDCl_3$):** δ 8.70 (d, 1H, 2.1 Hz, $H_2$); 8.02 (dd, 1H, 8.7 Hz and 2.1 Hz, $H_4$); 6.86 (d, 1H, 8.7 Hz, $H_5$); 4.64 (dd, 1H, 6.6 Hz and 3.3 Hz, $H_{2'}$); 4.02 (s, 3H, $OCH_3$); 3.45 (mt, 1H, $H_{5',\alpha}$); 3.35 (mt, 1H, $H_{5',\beta}$); 2.28-2.19 (m, 2H, $H_{3',\alpha}$ and $H_{3',\beta}$); 2.18-2.12 (m, 2H, $H_{4',\alpha}$ and $H_{4',\beta}$).

**RMN $^{13}$C NMR (100 MHz, $CDCl_3$):** δ 166.9 ($C_6$); 147.9 ($C_2$); 137.5 ($C_4$); 127.0 ($C_3$); 117.8 (CN); 111.7 ($C_5$); 54.4 ($OCH_3$); 48.5 ($C_{2'}$); 47.3 ($C_{5'}$); 31.9 ($C_{3'}$); 24.7 ($C_{4'}$).

**MS:** CI+: $[M+H]^+$ = 268.0; ESI+: $[M+H]^+$ = 268.1.

**HRMS** (ESI+) calculated for C11H14N3O3S $[M+H]^+$ m/z = 268.0750 found 268.0747.

**HPLC:** IA; Heptane/$CH_2Cl_2$ 8/2; 1 mL.min$^{-1}$; 20°C; 236 nm: tr = 37.45 min and 42.99 min.

**IR ATR:** ν (cm$^{-1}$) 3076.34-3000.14 (=C-H); 2989.32-2853.41 (-C-H); 2244.30 (CN); 1309.18 ($SO_2$,as); 1126.57 ($SO_2$,s).

**Example 9**: *Compound **3cc:** preparation of (S)-1-((6-methoxypyridin-3-yl) sulfonyl)pyrrolidine-2-carbonitrile*

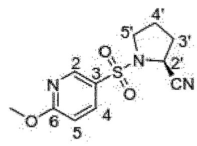

**Formula:** $C_{11}H_{13}N_3O_3S$

**Molecular weight:** 267.30 g.mol$^{-1}$

To a mixture of (S)-pyrrolidine-2-carbonitrile hydrochloride (40 mg, 0.30 mmol, 0.6 eq) in methylene chloride (10 mL) was added triethylamine (30.5mg, 0.04 mL, 0.30 mmol, 0.6 eq). The mixture was stirred during 15 min, 6-methoxypyridine-3-sulfonyl chloride (103.5 mg, 0.50 mmol, 1 eq) was added and the stirring was continued for additional 16 h. The mixture was then hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 62% yield.

**mp:** 109°C

**RMN $^1$H (300 MHz, $CDCl_3$):** δ 8.70 (d, 1H, 2.1 Hz, $H_2$); 8.02 (dd, 1H, 8.7 Hz and 2.1 Hz, $H_4$); 6.86 (d, 1H, 8.7 Hz, $H_5$); 4.64 (dd, 1H, 6.6 Hz and 3.3 Hz, $H_{2'}$); 4.02 (s, 3H, $OCH_3$); 3.45 (mt, 1H, $H_{5',\alpha}$); 3.35 (mt, 1H, $H_{5',\beta}$); 2.28-2.19 (m, 2H, $H_{3',\alpha}$ and $H_{3',\beta}$); 2.18-2.12 (m, 2H, $H_{4',\alpha}$ and $H_{4',\beta}$).

**RMN $^{13}$C (75 MHz, $CDCl_3$):** δ 167.1 ($C_6$); 148.1 ($C_2$); 137.7 ($C_4$); 127.2 ($C_3$); 118.0 (CN); 111.9 ($C_5$); 54.6 ($OCH_3$); 48.7 ($C_{2'}$); 47.4 ($C_{5'}$); 32.1 ($C_{3'}$); 24.8 ($C_{4'}$).

**MS:** ESI$^+$: $[M+H]^+$ = 268.1; $[M+Na]^+$ = 290.1.

**$[\alpha]_D^{20°C}$**: -100.8 (0,5 g/100 mL) in MeOH.

**HPLC:** IA; Heptane/$CH_2Cl_2$ 8/2; 1 mL.min$^{-1}$; 20°C; 236 nm: tr = 48.47 min.

**IR ATR:** ν (cm$^{-1}$) 3076.64-3001.90 (=C-H); 2991.53-2853.92 (-C-H); 2243.05 (CN); 1309.51 ($SO_2$,as); 1127.58 ($SO_2$,s).

**Example 10**: *Compound **3da:** preparation of 2-chloro-5-(pyrrolidin-1-ylsulfonyl)pyridine*

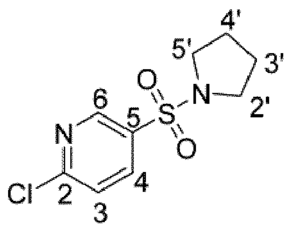

**Formula:** $C_9H_{11}ClN_2O_2S$
**Molecular weight:** 246.71 $g.mol^{-1}$

To a solution of 6-chloropyridine-3-sulfonyl chloride (150 mg, 0.71 mmol, 1 eq) in methylene chloride (10 mL) were added pyrrolidine (75.5 mg, 0.09 mL, 1.06 mmol, 1.5 eq) and triethylamine (107.4 mg, 0.15 mL, 1.06 mmol, 1.5 eq). The mixture was stirred during 16 h, hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid 69% yield.
**mp:** 134°C
**$^1$H NMR (400 MHz, $CDCl_3$):** $\delta$ 8.80 (d, 1H, 2.7 Hz, $H_6$); 8.04 (dd, 2H, 8.4 Hz and 2.7 Hz, $H_4$); 7.48 (d, 1H, 8.4 Hz, $H_3$); 3.27-3.24 (m, 4H, $H_{2',\alpha}$, $H_{2',\beta}$, $H_{5',\alpha}$ and $H_{5',\beta}$); 1.84-1.78 (m, 4H, $H_{3',\alpha}$, $H_{3',\beta}$, $H_{4',\alpha}$ and $H_{4',\beta}$).
**$^{13}$CNMR (100 MHz, $CDCl_3$):** $\delta$ 155.4 ($C_2$); 148.4 ($C_6$); 137.5 ($C_4$); 133.0 ($C_5$); 124.7 ($C_3$); 48.0 (2C, $C_{2'}$ and $C_{5'}$); 25.3 (2C, $C_{3'}$ and $C_{4'}$).
**MS:** ESI+: $[M+H]^+$ = 247.0; $[M+Na]^+$ = 269.0.
**HRMS** (ESI+) calculated for $C_9H_{12}ClN_2O_2S$ $[M+H]^+$ m/z = 247.0303 found 247.0294.
**IR ATR:** ν ($cm^{-1}$) 3087.05-3065.54 (=C-H); 2983.25-2857.84 (-C-H); 1346.02 ($SO_2$,as); 1161.98 ($SO_2$,s).

**Example 11**: *Compound **3db:** preparation of 1-((6-chloropyridin-3-yl)sulfonyl)pyrrolidine-2-carbonitrile*

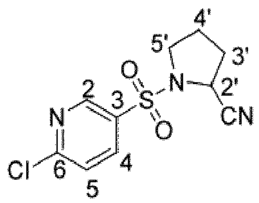

**Formula:** $C_{10}H_{10}ClN_3O_2S$
**Molecular weight:** 271.72 $g.mol^{-1}$

To a solution of pyrrolidine-2-carbonitrile (72.3 mg, 0.75 mmol, 1.1 eq) in methylene chloride (10 mL) were added triethylamine (138 mg, 0.20 mL, 1.37 mmol, 2 eq) and 6-chloropyridine- 3-sulfonyl chloride (145 mg, 0.68 mmol, 1 eq). The mixture was stirred during 2 days then hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 59% yield.
**mp:** 128°C
**RMN $^1$H NMR (400 MHz, $CDCl_3$):** $\delta$ 8.89 (d, 1H, 2.1 Hz, $H_2$); 8.16 (dd, 2H, 8.4 Hz and 2.1 Hz, $H_4$); 7.53 (d, 1H, 8.4 Hz, $H_5$); 4.71 (t, 1H, 5.4 Hz, $H_{2'}$); 3.52 (mt, 1H, $H_{5',\alpha}$); 3.35 (m, 1H, $H_{5',\beta}$); 2.31-2.25 (m, 2H, $H_{3',\alpha}$ and $H_{3',\beta}$); 2.17-2.12 (m, 2H, $H_{4',\alpha}$ and $H_{4',\beta}$).
**RMN $^{13}$C NMR (100 MHz, $CDCl_3$):** $\delta$ 156.4 ($C_3$); 148.6 ($C_2$); 137.6 ($C_4$); 133.5 ($C_6$); 125.0 ($C_5$); 117.3 (CN); 48.6 ($C_{2'}$); 47.4 ($C_{5'}$); 31.9 ($C_{3'}$); 24.7 ($C_{4'}$).
**MS:** CI+: $[M]^{+\bullet}$ = 271.4; $[M+NH_4]^+$ = 289.0; ESI+: $[M+H]^+$ = 272.0 $[M+Na]^+$ = 294.0.
**HRMS** (ESI+) calculated for $C_{10}H_{10}ClN_3NaO_2S$ [M+Na+] m/z = 294.0074 found 294.0075.
**HPLC:** IA; $CH_2Cl_2$; 0.5 $mL.min^{-1}$; 20°C; 240 nm: tr = 6.99 min and 8.57 min.
**IR ATR:** ν ($cm^{-1}$) 3086.46-3037.94 (=C-H); 2994.57-2896.65 (-C-H); 2242.63 (CN); 1354.03 ($SO_2$,as); 1164.39 ($SO_2$,s).

**Example 12**: *Compound **3dc:** preparation of (S)-1-((6-chloropyridin-3-yl)sulfonyl)pyrrolidine-2- carbonitrile*

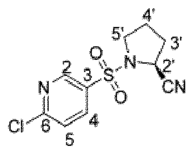

**Formula:** $C_{10}H_{10}ClN_3O_2S$
**Molecular weight:** 271.72 g.mol$^{-1}$

To a mixture of (S)-pyrrolidine-2-carbonitrile hydrochloride (103 mg, 0.79 mmol, 1.1 eq) in methylene chloride (10 mL) was added triethylamine (143 mg, 0.2 mL, 1.41 mmol, 2 eq). The mixture was stirred during 15 min, 6-chloropyridine-3-sulfonyl chloride (150 mg, 0.71 mmol, 1 eq) was added and the stirring was continued for additional 16 h. The mixture was then hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid 67% yield.

**mp:** 128°C

**$^1$H NMR (300 MHz, CDCl$_3$):** $\delta$ 8.89 (d, 1H, 2.1 Hz, $H_2$); 8.16 (dd, 2H, 8.4 Hz and 2.1 Hz, $H_4$); 7.54 (d, 1H, 8.4 Hz, $H_5$); 4.70 (t, 1H, 5.4 Hz, $H_{2'}$); 3.52 (mt, 1H, $H_{5',\alpha}$); 3.35 (m, 1H, $H_{5',\beta}$); 2.31-2.25 (m, 2H, $H_{3',\alpha}$ and $H_{3',\beta}$); 2.18-2.10 (m, 2H, $H_{4',\alpha}$ and $H_{4',\beta}$).

**13C NMR (75 MHz, CDCl$_3$):** $\delta$156.4 ($C_6$); 148.6 ($C_2$); 137.7 ($C_4$); 133.5 ($C_3$); 125.0 ($C_5$); 117.3 (CN); 48.6 ($C_{2'}$); 47.4 ($C_{5'}$); 31.9 ($C_{3'}$); 24.7 ($C_{4'}$).

**MS:** ESI+: $[M+H]^+$ = 272.0; $[M+Na]^+$ = 294.0.

**HRMS** (ESI+) calculated for $C_{10}H_{11}ClN_3O_2S$ $[M+H]^+$ m/z = 272.0255 found 272.0251.

**$[\alpha]_D^{20°C}$**: -89.9 (1 g/100 mL) in MeOH.

**HPLC:** IA; $CH_2Cl_2$; 0.5 mL.min$^{-1}$; 20°C; 240 nm: tr = 7.25 min.

**IR ATR:** ν (cm$^{-1}$) 3088.54-3001.24 (=C-H); 2986.01-2878.28 (-C-H); 2246.46 (CN); 1352.87 ($SO_2$,as); 1165.28 ($SO_2$,s).

**Example 13:** *Compound **3ec**: preparation of (S)-1-((6-methylpyridin-3-yl)sulfonyl)pyrrolidine-2- carbonitrile*

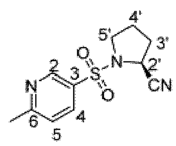

**Formula:** $C_{11}H_{13}N_3O_2S$
**Molecular weight:** 251.30 g.mol$^{-1}$

To a mixture of (S)-pyrrolidine-2-carbonitrile hydrochloride (32.5 mg, 0.25 mmol, 1.1 eq) in methylene chloride (10 mL) was added triethylamine (45 mg, 0.06 mL, 0.45 mmol, 2 eq). The mixture was stirred during 15 min, 6-methylpyridine-3-sulfonyl chloride (42.7 mg, 0.22 mmol, 1 eq) was added and the stirring was continued for additional 16 h. The mixture was then hydrolyzed with an aqueous solution of HCl (2M) and extracted with methylene chloride. The organic layer was washed with water, dried over $MgSO_4$, filtrated and evaporated under vacuum. The crude product was purified by column chromatography ($SiO_2$, petroleum ether/EtOAc: 6/4) to afford the product as a white solid in 70% yield.

**mp:** 120°C

**RMN $^1$H NMR (400 MHz, CDCl$_3$):** $\delta$ 8.98 (d, 1H, 2.1 Hz, $H_2$); 8.08 (dd, 2H, 8.4 Hz and 2.1 Hz, $H_4$); 7.35 (d, 1H, 8.4 Hz, $H_5$); 4.66 (dd, 1H, 7.1 Hz and 3.2 Hz, $H_{2'}$); 3.48 (mt, 1H, $H_{5',\alpha}$); 3.36 (m, 1H, $H_{5',\beta}$); 2.28-2.19 (m, 2H, $H_{3',\alpha}$ and $H_{3',\beta}$); 2.15-2.06 (m, 2H, $H_{4',\alpha}$ and $H_{4',\beta}$).

**RMN $^{13}$C NMR (100 MHz, CDCl$_3$):** 164.2 ($C_6$); 147.7 ($C_2$); 135.4 ($C_4$); 131.6 ($C_3$); 123.5 ($C_5$); 117.6 (CN); 48.6 ($C_{2'}$); 47.3 ($C_{5'}$); 31.9 ($C_{3'}$); 27.8 ($CH_3$); 24.7 ($C_{4'}$).

**MS:** ESI+: $[M+H]^+$ = 252.1; $[M+Na]^+$ = 274.1.

**HRMS** (ESI+) calculated for $C_{11}H_{13}N_3NaO_2S$ $[M+Na]^+$ m/z = 274.0626 found 274.0631.

**$[\alpha]_D^{20°C}$**: -93.3 (0.5 g/100 mL) in MeOH.

**IR ATR:** ν (cm$^{-1}$) 3065.82-2995.28 (=C-H); 2939.80-2852.41 (-C-H); 2245.49 (CN); 1341.03 ($SO_2$,as); 1162.68 ($SO_2$,s).

## ELECTROPHYSIOLOGY AND INSECT TOXICOLOGICAL STUDIES

### NEUROTOXIC EFFECT ON INSECT SYNAPTIC TRANSMISSION

***Material and Methods***

Neurotoxic effect was studied using mannitol-gap recordings. Experiments were performed on the cercal nerve giant interneuron synapses located within the cockroach sixth abdominal ganglion (A6) using the mannitol-gap method pioneered by Callec (Callec, J. J.; Sattelle, D. B., A simple technique for monitoring the synaptic actions of pharmacological agents. J Exp Biol 1973, 59, (3), 725-38; and Callec, J. J.; Sattelle, D. B.; Hue, B.; Pelhate, M., Central synaptic actions of pharmacological agents in insects: oil-gap and mannitol-gap studies. In Neurotox 79, Sherwood, M., Ed. Plenum Press: New York, 1980; pp 93-100).

Electrical events were recorded using external electrodes. A non-electrolyte medium (mannitol) was interposed between the recording sites (Callec, J.J.; Sattelle, D.B.; Hue, B.; Pelhate, M., Central synaptic actions of pharmacological agents in insects: oil-gap and mannitol-gap studies. In Neurotox 79, Sherwood, M., Ed. Plenum Press: New York, 1980; pp 93-100). The main advantages of this method were to preserve the recordings of the unitary or evoked excitatory postsynaptic potentials (EPSP) and the postsynaptic polarization. Consequently, monitoring the variations of EPSP amplitude and/or polarization induced by drug application enables dose-response curves to be recorded. Moreover, this set-up allows long-term experiments to be performed and test solutions can be readily applied without any of the technical problems associated with intracellular recording.

A6 was carefully desheathed to facilitate penetration of bath-applied drugs. The recording electrodes were connected to the input of high-impedance amplifier, whose outputs were passed to a numeric oscilloscope (Hameg, Germany) and a chart recorder (Kipp and Zonen, BD 111, Holland). Variation of postsynaptic polarization was monitored on a chart recorder and the cEPSPs were evoked by electrical stimulations of the ipsilateral cercal nerve XI using a dual pulse stimulator (Campden 915, USA). TMX was applied during 3 min in the same conditions as previously published (Buckingham, S.; Lapied, B.; Corronc, H.; Sattelle, F., Imidacloprid actions on insect neuronal acetylcholine receptors. J Exp Biol 1997, 200, (Pt 21), 2685-92; and Thany, S. H., Agonist actions of clothianidin on synaptic and extrasynaptic nicotinic acetylcholine receptors expressed on cockroach sixth abdominal ganglion. Neurotoxicology 2009, 30, (6), 1045-52), with a micropump fast perfusion (Harvard Apparatus) that produced a constant solution exchange (500 $\mu$L/min). All muscarinic and nicotinic antagonists were bath-applied for at least 20 min before a single application of TMX. Recordings were made at room temperature.

To compare the depolarizations, the peak amplitudes were normalized (V/Vmax). The dose-response curve was derived from the fitted curve following the equation:

$$y = V_{min} + (V_{max} - V_{min}) / (1 + 10^{(\log EC_{50} - X)H})$$

where Y is the normalized response, $V_{max}$ and $V_{min}$ are the maximum and minimum responses, H is the Hill coefficient, $EC_{50}$ is the concentration giving half the maximum response and X is the logarithm of the compound concentration.

***Results***

The following compounds, **3i-3iv**, all derivatives of compound 3 of Tables 1 and 2 of the Molecular Modeling section, were tested for their toxicity on insect synaptic transmission and their toxic effect on honeybees.

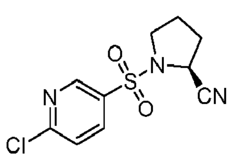
**3i**

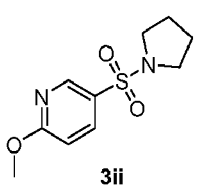
**3ii**

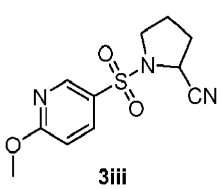
**3iii**

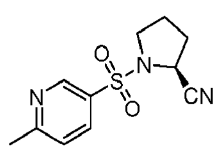
**3iv**

Compounds 3i, 3ii, 3iii and 3iv correspond to examples 12, 7, 8, and 13, respectively, described in the chemistry section.

The neurotoxicity of the four compounds on insect cholinergic synaptic transmission was examined. Indeed, cockroach cercal afferent giant interneuron synapse was used as a model to study the neurotoxicity of neonicotinoid insecticides on cockroach cholinergic synaptic transmission. Thus, the aim was to demonstrate that these compounds were able to depolarize the sixth abdominal ganglion as found with neonicotinoid insecticides, in particular, imidacloprid

(IMI) the forerunner of neonicotinoid insecticides (Buckingham, S.D.; Lapied, B.; LeCorronc, H.; Grolleau, F.; Sattelle, D.B., Imidacloprid actions on insect neuronal acetylcholine receptors. J. Exp. Biol. 1997, 200, (21), 2685-2692; and Thany, S. H., Agonist actions of clothianidin on synaptic and extrasynaptic nicotinic acetylcholine receptors expressed on cockroach sixth abdominal ganglion. Neurotoxicology 2009, 30, (6), 1045-1052).

Bath application of the four compounds **3i**, **3ii**, **3iii** and **3iv** induced a strong depolarization (Figures 1, 2, 3, and 4) of the sixth abdominal ganglion. Their effect on synaptic depolarization was not reversed after wash-out and this effect was also observed 2h after application. By recording the depolarization, a dose-response curve was plotted according to equation described in Materials and methods section.

The $EC_{50}$ values were 19 μM, 8.02 μM and 4.25 μM for compound **3i**, **3ii** and **3iii** respectively.

Note that, the EC50 value of imidacloprid was around 15 ± 0.12 μM. Thus, among the series, the most effective compounds appear to be **3ii** and **3iii**, with an $EC_{50}$ value of 8.02 μM and 4.25 μM. In addition, the **3ii** induced depolarization was not reversed after wash-out and this effect was observed 2 h after application.

## TOXICITY AGAINST HONEYBEE APIS MELLIFERA

### *Material and Methods*

### 1. Honeybee colonies

Honeybees were kept from hives located at the University of Orleans and transferred to the laboratory incubators for experiments (30 ± 1.5°C; 12/12h O/N). They were placed in six different cages, 20 bees per cage, with 40% sucrose solution (control group) or tested compounds.

(1) Cage size: 20 x 20 x 13 cm

All tests were carried out using adult worker honeybees, *Apis mellifera L* (Hymenoptera: Apidae) taken from two queen colonies. These colonies were disease-free and had received no chemical treatments (Eg. Varroacide). For oral toxicity tests, adult worker bees were collected from the hive combs (avoiding the brood nest area) or from the flight board.

### 2. Test concentrations

Stock solutions of compounds were dissolved in dimethyl sulfoxide (DMSO) to obtain 500 ng/μL and stocked at -20°C. These samples were then diluted in sucrose solution (40%; w/v), for final experiments. The final solution for each test and control solution contained less than 0.25% of DMSO and were stocked at 4°C. Solutions of the test doses were stirred immediately prior to use and were visually homogenous when proposed to bees. Bee allocation to the treatment groups was made impartially. Before being used for the test, bees were subjected to a starvation period of between 2h and 3h under test conditions (For review, see Nauen, R.; Ebbinghaus-Kintscher, U.; Schmuck, R., Toxicity and nicotinic acetylcholine receptor interaction of imidacloprid and its metabolites in Apis mellifera (Hymenoptera : Apidae). Pest Management Science 2001, 57, (7), 577-586).

**Table 3.** Concentrations used to evaluate the toxic effect of neonicotinoid insecticides against Honey bees *Apis mellifera.*

| Compounds | $LD_{50}$ |
|---|---|
| Nitenpyram | 138 ng/bee<br>(Iwasa, T.; Motoyama, N.; Ambrose, J. T.; Roe, R. M., Mechanism for the differential toxicity of neonicotinoid insecticides in the honey bee, Apis mellifera. Crop Protection 2004, 23, (5), 371-378) |
| Dinotefuran | 75 ng/bee (Iwasa 2004) |
| Clothianidin | 21.8 ng/bee (Iwasa 2004) |
| Thiamethoxam | 29.9 ng/bee (Iwasa 2004) |
| Imidacloprid | 6.7-23.8 ng/bee<br>(Stark, J. D.; Jepson, P. C.; Mayer, D. F., Limitations to use of topical toxicity data for predictions of pesticide side-effects in the field. Journal of Economic Entomology 1995, 88, (5), 1081-1088) 17,9 ng/bee (Iwasa 2004) |
| Thiacloprid | 24.2 μg/bee |

(continued)

| Compounds | $LD_{50}$ |
|---|---|
| | (Elbert, A.; Erdelen, C.; Kuhnhold, J.; Nauen, R.; Schmidt, H. U.; Hattori, Y.; Bcpc; Bcpc, Thiacloprid, a novel neonicotinoid insecticide for foliar application. In Bcpc Conference: Pests & Diseases 2000, Vols 1-3, Proceedings, 2000; Vol. 1-3, pp 21-26) 14.6 ng/bee (Iwasa 2004) |
| Acetamiprid | 7.1 μg/bee<br>7.07 μg/bee (Iwasa 2004) |

Six batches of bees (20 bees per batch) were subjected to different doses of the test compound (nominal doses between 50 ng/bee and 159 ng/bee). Doses were determined according to the lethal dose of neonicotinoid insecticides inducing bee toxic effects (Table 3). Controls received sucrose solution containing 0.25% DMSO. The glass test feeders containing any unconsumed portions of the doses were removed and after each experiment a fresh sucrose solution was supplied in feeders within the cages. For each concentration three replicates were made (180 bees/concentration). Mortality was measured at 24 h and 48 h after treatment.

***Results***

Compounds **3i-3iv** were tested for their toxic effect on honeybees.

Two concentrations were evaluated, estimated according to neonicotinoid concentrations used to evaluate bee toxic effect. For imidacloprid, the LD50 tested was ranged between 6.7 ng/bee and 23.8 ng/bee but several neonicotinoids, such as thiacloprid and acetamiprid, were estimated to have an $LD_{50}$ value more than 7 μg/bee.

To be sure that these compounds are not able to induce strong toxic effect on bees, the concentrations 50 ng/bee and 150 ng/bee were used. Interestingly, none of these compounds induced a strong mortality of honey bees after oral application, (Table 4).

**Table 4.** Toxic effects on bees of four compounds according to the invention.

| Compounds | | **Concentration** | **% mortality** | |
|---|---|---|---|---|
| | | | 24 h | 48 h |
| - | Control | DMSO 1% | 0 | 0 |
| **3i** | DMSO 1% | 50 ng/bee | 3.3 | 10.0 |
| | | 150 ng/bee | 0 | 0 |
| **3ii** | DMSO 1% | 50 ng/bee | 0 | 3.3 |
| | | 150 ng/bee | 0 | 1.2 |
| **3iii** | DMSO 1% | 50 ng/bee | 3.3 | 5.0 |
| | | 150 ng/bee | 2.5 | 3.7 |
| **3iv** | DMSO 1% | 50 ng/bee | 0 | 0 |
| | | 150 ng/bee | 0 | 0 |

Thus, these compounds are more toxic on the cockroach nervous system than imidacloprid. Furthermore, two of them (**3ii** and **3iv**), appeared not toxic for bees because at higher concentrations (150 ng/bee), they were unable to induce toxicity. Moreover, in general, the toxic effect observed was under 10% mortality for all compounds.

In each case, n = 60 bees and experiments were triplicates. Data from control conditions were pooled because not significant effect of 1% DMSO was found on bees mortality.

## Claims

**1.** A compound having the following formula (I):

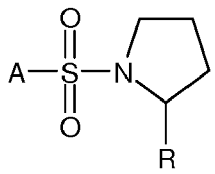

(I)

wherein:

- A is a (hetero)aryl radical comprising from 5 to 10 carbon atoms, possibly substituted by at least one substituent chosen from the group consisting of: halogen atoms, amino, azido, cyano, nitro, hydroxyl, formyl, carboxyl, amido, $(C_1-C_6)$alkyl groups, halo$(C_1-C_6)$alkyl groups, $(C_1-C_6)$alkoxy groups, alkenyl groups, cycloalkenyl groups, and alkynyl groups, and
- R is H, CN or $CF_3$,

or their pharmaceutically acceptable salts, racemates, diastereomers or enantiomers.

**2.** The compound of claim 1, wherein A is a phenyl group, possibly substituted by at least a substituent chosen from the group consisting of: halogen atoms, amino, azido, cyano, nitro, hydroxyl, formyl, carboxyl, amido, $(C_1-C_6)$alkyl groups, halo$(C_1-C_6)$alkyl groups, $(C_1-C_6)$alkoxy groups, alkenyl groups, cycloalkenyl groups, and alkynyl groups.

**3.** The compound of claim 1, wherein A is a heteroaryl group comprising from 5 to 10 atoms including 1 to 4 heteroatoms selected from O, S or N, possibly substituted by at least a substituent chosen from the group consisting of: halogen atoms, amino, azido, cyano, nitro, hydroxyl, formyl, carboxyl, amido, $(C_1-C_6)$alkyl groups, halo$(C_1-C_6)$alkyl groups, $(C_1-C_6)$alkoxy groups, alkenyl groups, cycloalkenyl groups, and alkynyl groups.

**4.** The compound of claim 1, having the following formula (II):

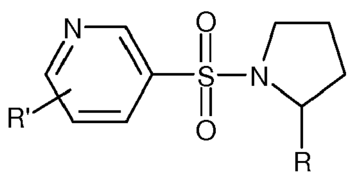

(II)

wherein:

- R is as defined in claim 1; and
- R' is a substituent chosen from the group consisting of: halogen atoms, amino, azido, cyano, nitro, hydroxyl, formyl, carboxyl, amido, $(C_1-C_6)$alkyl groups, halo$(C_1-C_6)$alkyl groups, $(C_1-C_6)$alkoxy groups, aminoalkyl groups, alkenyl groups, cycloalkenyl groups, and alkynyl groups.

**5.** The compound of claim 4, having the following formula (III):

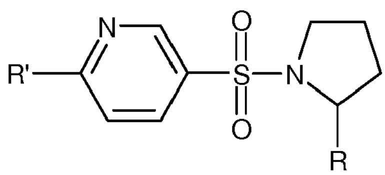

(III)

wherein:

- R is as defined in claim 1; and
- R' is a substituent chosen from the group consisting of: halogen atoms, $(C_1-C_6)$alkyl groups, and $(C_1-C_6)$alkoxy groups, preferably Cl, $OCH_3$ or $CH_3$.

**6.** The compound of any of claims 1 to 5, selected from the followings:

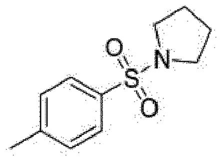 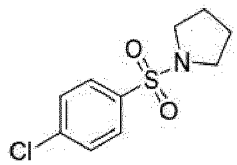

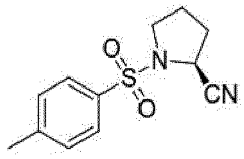 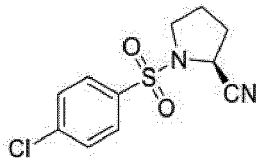

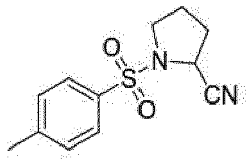 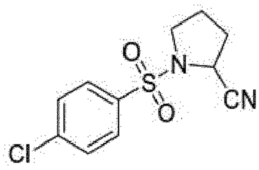

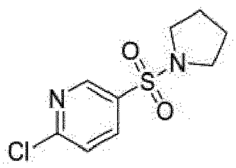 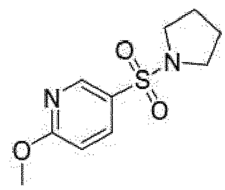

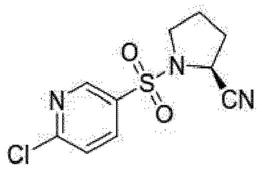 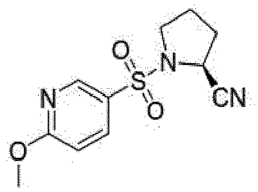 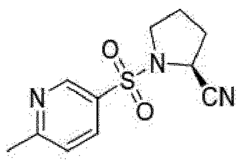

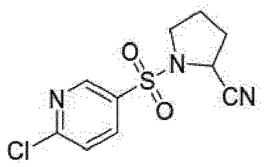 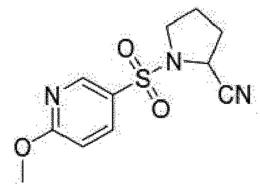

7. A process for preparing a compound of any one of claims 1 to 6, comprising the reaction between a compound having the following formula (IV):

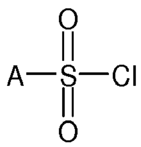

(IV)

A being as defined above in any one of claims 1 to 3, with a compound having the following formula (V):

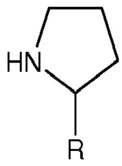

(V)

R being as defined above in claim 1.

**8.** An agrochemical composition, in particular a pesticide composition (e.g. acaricides, insecticides, nematicides, molluscicides), comprising at least one compound of any one of claims 1 to 6.

**9.** The use of a compound as defined in any one of claims 1 to 6, as an insecticide.

**10.** The use of claim 9, wherein the compound has the formula (III) of claim 6.

**11.** The use of claim 9 or 10, wherein the compound is chosen from the following compounds:

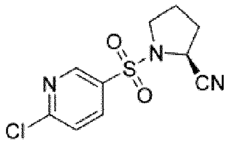 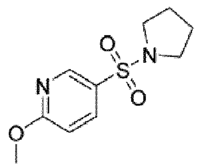 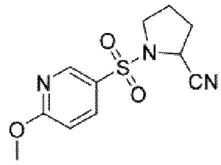 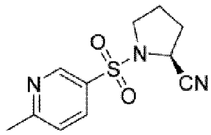

**12.** A method for pest control in plants or for animal welfare, comprising the application of at least one compound as defined in any one of claims 1 to 6 to a plant, a plant seed, a plant part, fruit or an animal skin.

**13.** The method of claim 12, wherein the compound has the formula (III) of claim 6.

**14.** The method of claim 12 or 13, wherein the compound is chosen from the following compounds:

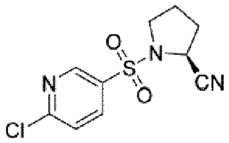 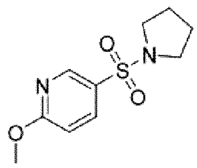 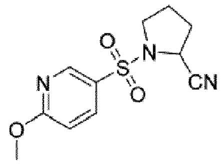 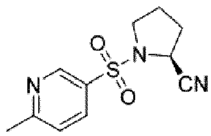

**15.** A compound of any one of claims 1 to 6, for its use in the prevention of vector-borne diseases.

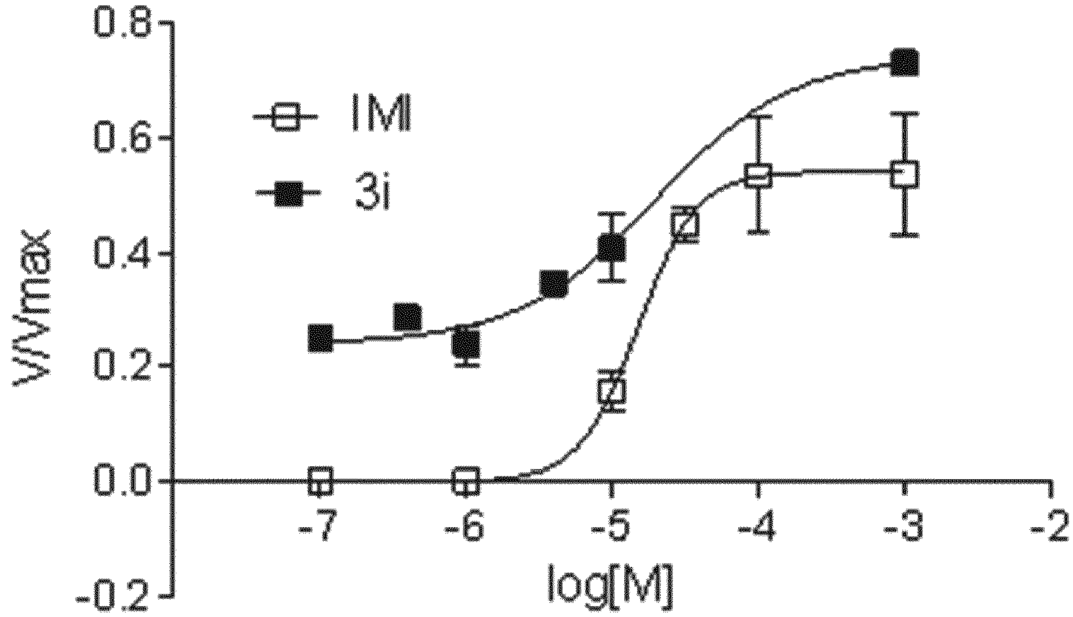
0.8
0.6
0.4
0.2
0.0
-0.2
V/Vmax
IMI
3i
-7
-6
-5
-4
-3
-2
log[M]

FIG.1

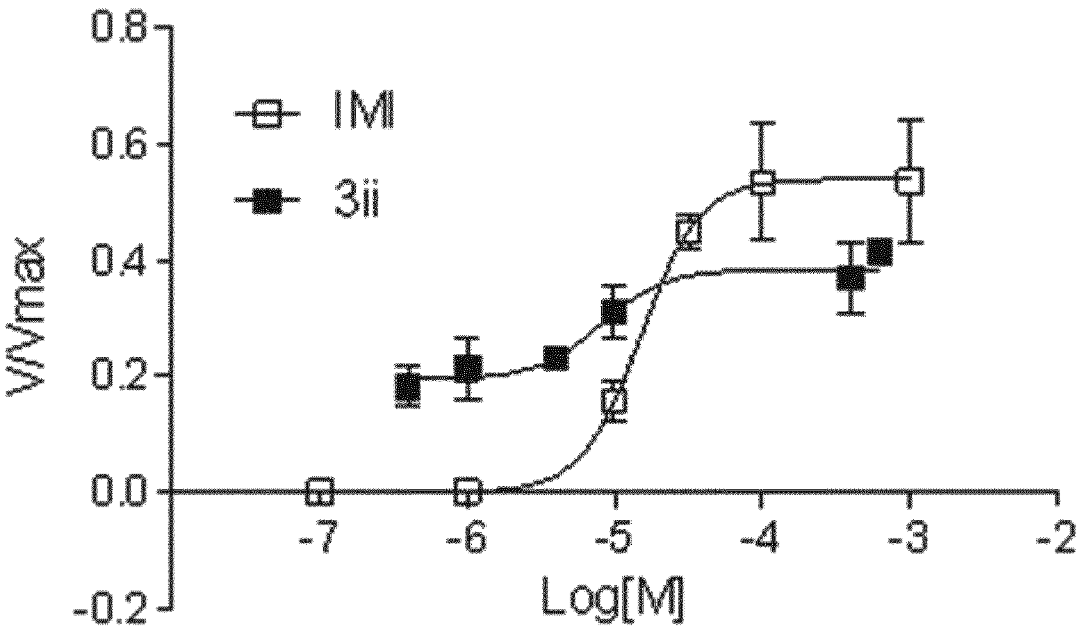
0.8
0.6
0.4
0.2
0.0
-0.2
V/Vmax
IMI
3ii
-7
-6
-5
-4
-3
-2
Log[M]

FIG.2

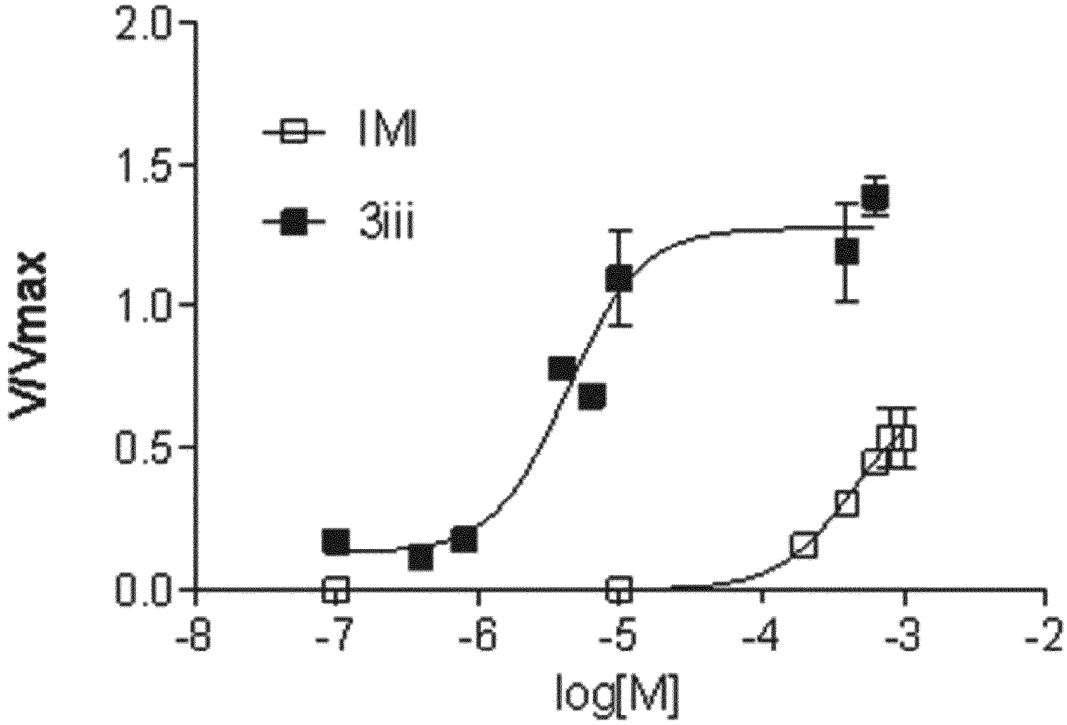
2.0
1.5
1.0
0.5
0.0
V/Vmax
IMI
3iii
-8
-7
-6
-5
-4
-3
-2
log[M]

FIG.3

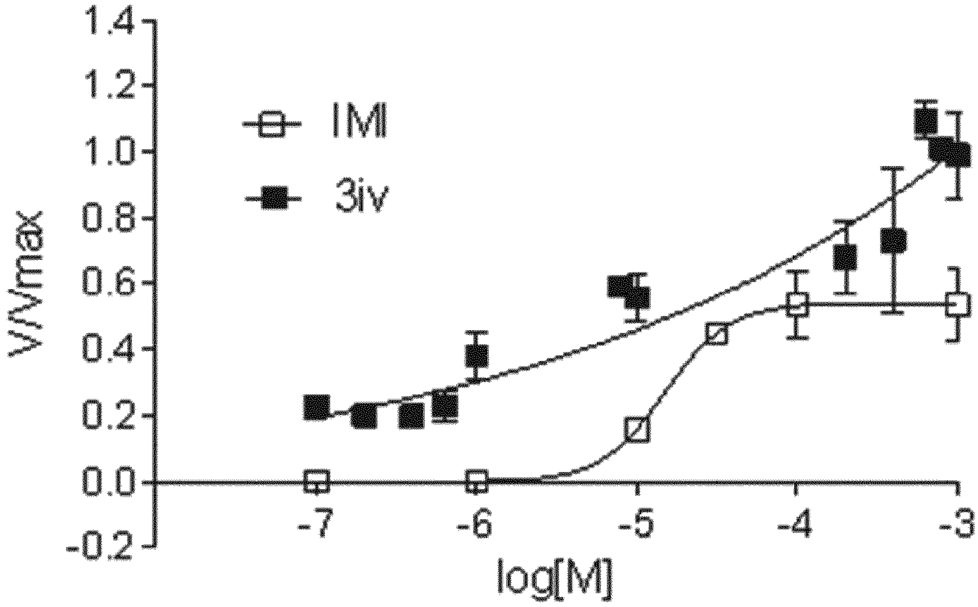
1.4
1.2
1.0
0.8
0.6
0.4
0.2
0.0
-0.2
V/Vmax
IMI
3iv
-7
-6
-5
-4
-3
log[M]

FIG.4

Europäisches Patentamt
European Patent Office
Office européen des brevets

# EUROPEAN SEARCH REPORT

Application Number
EP 18 30 6468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/105748 A1 (BAYER CROPSCIENCE GMBH [DE]; BASTIAANS HERNICUS M M [DE]; KRAUTSTRUNK) 10 November 2005 (2005-11-10) * claims 1,4,5; example 69 * | 1,3,4, 7-9,12, 15 | INV. C07D401/12 A01N43/36 C07D207/48 A61P31/04 |
| X | US 4 379 157 A (VAN HES ROELOF [NL] ET AL) 5 April 1983 (1983-04-05) * column 7, line 44 - column 8, line 35; example I; table A; compound 27 * | 1,2,8,9, 12,15 | |
| X | EP 0 433 001 A1 (ICI PLC [GB]) 19 June 1991 (1991-06-19) * scheme I, p.24; example 4; compounds 5,18,39,52,73,86 * | 1-15 | |
| X | WO 2014/146493 A1 (MERCK SHARP & DOHME [US]; DINSMORE CHRISTOPHER [US]; FULLER PETER [US]) 25 September 2014 (2014-09-25) * intermediates I-18 and I-35 and their preparation on p.50; pages 50-54 * | 1,2,7 | |
| | | | TECHNICAL FIELDS SEARCHED (IPC) |
| X | WO 2007/092558 A2 (WYETH CORP [US]; GOPALSAMY ARIAMALA [US]) 16 August 2007 (2007-08-16) * claims 1,3,7,8,13; examples 2,23 * | 1,2,7 | C07D A01N A61P |
| X | WO 99/45006 A1 (PFIZER [US]; PFIZER LTD [GB]; BULL DAVID JOHN [GB]; MAGUIRE ROBERT JOH) 10 September 1999 (1999-09-10) * preparation 51; page 156 * | 1,2 | |

-/--

The present search report has been drawn up for all claims

2

| Place of search | Date of completion of the search | Examiner |
|---|---|---|
| Munich | 21 March 2019 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 30 6468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VERENA HUGENBERG ET AL: "Oxidative desulfurization-fluorination of thioethers. Application for the synthesis of fluorinated nitrogen containing building blocks", ORGANIC & BIOMOLECULAR CHEMISTRY, vol. 8, no. 24, 1 January 2010 (2010-01-01), page 5682, XP055571707, ISSN: 1477-0520, DOI: 10.1039/c0ob00560f * compound 7a * ----- | 1,2 | |
| X | SHARMA RADHIKA ET AL: "Design and synthesis of sulfonamide derivatives of pyrrolidine and piperidine as anti-diabetic agents", EUROPEAN JOURNAL OF MEDICINAL CHEMISTRY, EDITIONS SCIENTIFIQUE ELSEVIER, PARIS, FR, vol. 90, 22 November 2014 (2014-11-22), pages 342-350, XP029125969, ISSN: 0223-5234, DOI: 10.1016/J.EJMECH.2014.11.041 * compound 9b * ----- | 1,2 | TECHNICAL FIELDS SEARCHED (IPC) |
| X | KARL HEMMING ET AL: "Azide based routes to tetrazolo and oxadiazolo derivatives of pyrrolobenzodiazepines and pyrrolobenzothiadiazepines", TETRAHEDRON, vol. 70, no. 40, 1 October 2014 (2014-10-01), pages 7306-7317, XP055571730, AMSTERDAM, NL ISSN: 0040-4020, DOI: 10.1016/j.tet.2014.07.050 * compound 28b * ----- -/-- | 1,2 | |

The present search report has been drawn up for all claims

2

| Place of search | Date of completion of the search | Examiner |
|---|---|---|
| Munich | 21 March 2019 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 30 6468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOSHIHIRO INAMOTO ET AL: "Indium Triiodide Catalyzed Reductive Functionalization of Amides via the Single-Stage Treatment of Hydrosilanes and Organosilicon Nucleophiles", ORGANIC LETTERS, vol. 15, no. 13, 5 July 2013 (2013-07-05), pages 3452-3455, XP055571746, US ISSN: 1523-7060, DOI: 10.1021/ol4015317 * scheme 1; compound 3o * | 1,2,6 | |
| X | Doreen M Ashworth ET AL: "2-cyanopyrrolidides as potent, stable inhibitors of dipeptidyl peptidase IV", Bioorganic & Medicinal Chemistry Letters, 1 January 1996 (1996-01-01), pages 1163-1166, XP055571760, DOI: 10.1016/0960-894X(96)00190-4 Retrieved from the Internet: URL:https://www.sciencedirect.com/science/article/pii/0960894X96001904/pdf?md5=2f09a7614a149e8d99d4ddd444919c42&pid=1-s2.0-0960894X96001904-main.pdf * compound 3 in scheme I * | 1,2 | TECHNICAL FIELDS SEARCHED (IPC) |
| X | WO 2008/002974 A1 (ABBOTT LAB [US]; FAGHIH RAMIN [US]; GFESSER GREGORY A [US]; LYNCH CHRI) 3 January 2008 (2008-01-03) * 3-(pyrrolidine-1-sulfonyl)-phenylamins starting material in example 22, p.55 * | 1,2 | |
| A | WO 2016/014847 A1 (UNIV NORTHEASTERN [US]; UNIV FLORIDA [US]) 28 January 2016 (2016-01-28) * examples 56 and 57 on p.36; claims 14-17 * | 1,2 | |

The present search report has been drawn up for all claims

2

| Place of search | Date of completion of the search | Examiner |
|---|---|---|
| Munich | 21 March 2019 | Schuemacher, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 18 30 6468

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2019

| Patent document cited in search report | Publication date | Patent family member(s) | Publication date |
|---|---|---|---|
| WO 2005105748 A1 | 10-11-2005 | DE 102004018953 A1 | 17-11-2005 |
| | | WO 2005105748 A1 | 10-11-2005 |
| US 4379157 A | 05-04-1983 | AR 226871 A1 | 31-08-1982 |
| | | AU 6636081 A | 30-07-1981 |
| | | BR 8100281 A | 04-08-1981 |
| | | CA 1167043 A | 08-05-1984 |
| | | CS 219348 B2 | 25-03-1983 |
| | | DD 157071 A5 | 13-10-1982 |
| | | DE 3162624 D1 | 12-07-1984 |
| | | DK 24181 A | 24-07-1981 |
| | | EP 0033984 A1 | 19-08-1981 |
| | | ES 8200654 A1 | 01-11-1981 |
| | | ES 8201965 A1 | 01-01-1982 |
| | | GR 72782 B | 05-12-1983 |
| | | HU 186379 B | 29-07-1985 |
| | | IE 50835 B1 | 23-07-1986 |
| | | IL 61942 A | 29-11-1985 |
| | | JP S56120664 A | 22-09-1981 |
| | | NZ 196046 A | 12-04-1983 |
| | | PL 229285 A1 | 13-04-1982 |
| | | RO 81451 A | 02-04-1984 |
| | | SU 1093247 A3 | 15-05-1984 |
| | | US 4379157 A | 05-04-1983 |
| | | YU 14981 A | 31-10-1983 |
| | | ZA 8100382 B | 24-02-1982 |
| EP 0433001 A1 | 19-06-1991 | EP 0433001 A1 | 19-06-1991 |
| | | GB 2239017 A | 19-06-1991 |
| | | JP H04210965 A | 03-08-1992 |
| | | US 5137886 A | 11-08-1992 |
| | | ZA 9009661 B | 28-08-1991 |
| WO 2014146493 A1 | 25-09-2014 | AU 2014234909 A1 | 13-08-2015 |
| | | BR 112015024050 A2 | 18-07-2017 |
| | | CA 2901770 A1 | 25-09-2014 |
| | | CN 105121444 A | 02-12-2015 |
| | | EP 2976341 A1 | 27-01-2016 |
| | | JP 6305510 B2 | 04-04-2018 |
| | | JP 2016514711 A | 23-05-2016 |
| | | KR 20150132170 A | 25-11-2015 |
| | | RU 2015144385 A | 26-04-2017 |
| | | US 2016272634 A1 | 22-09-2016 |
| | | WO 2014146493 A1 | 25-09-2014 |
| WO 2007092558 A2 | 16-08-2007 | AU 2007212293 A1 | 16-08-2007 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 18 30 6468

This annex lists the patent family members relating to the patent documents cited in the above-mentioned European search report. The members are as contained in the European Patent Office EDP file on
The European Patent Office is in no way liable for these particulars which are merely given for the purpose of information.

21-03-2019

| Patent document cited in search report | Publication date | Patent family member(s) | Publication date |
|---|---|---|---|
| | | BR PI0707558 A2 | 10-05-2011 |
| | | CA 2640229 A1 | 16-08-2007 |
| | | CN 101379030 A | 04-03-2009 |
| | | EP 1981845 A2 | 22-10-2008 |
| | | JP 2009526065 A | 16-07-2009 |
| | | US 2007225344 A1 | 27-09-2007 |
| | | WO 2007092558 A2 | 16-08-2007 |
| WO 9945006 A1 | 10-09-1999 | AR 015528 A1 | 02-05-2001 |
| | | AT 248836 T | 15-09-2003 |
| | | AU 2181099 A | 20-09-1999 |
| | | BR 9908480 A | 05-12-2000 |
| | | CA 2322442 A1 | 10-09-1999 |
| | | CO 4790154 A1 | 31-05-1999 |
| | | DE 69910984 T2 | 15-07-2004 |
| | | DK 1060178 T3 | 24-11-2003 |
| | | EP 1060178 A1 | 20-12-2000 |
| | | ES 2204101 T3 | 16-04-2004 |
| | | GT 199900029 A | 19-08-2000 |
| | | JP 3668133 B2 | 06-07-2005 |
| | | JP 2002505329 A | 19-02-2002 |
| | | MA 26611 A1 | 20-12-2004 |
| | | PA 8468501 A1 | 29-09-2000 |
| | | PT 1060178 E | 31-12-2003 |
| | | TN SN99031 A1 | 10-11-2005 |
| | | US 6610707 B1 | 26-08-2003 |
| | | WO 9945006 A1 | 10-09-1999 |
| WO 2008002974 A1 | 03-01-2008 | AU 2007265116 A1 | 03-01-2008 |
| | | BR PI0712667 A2 | 04-09-2012 |
| | | CA 2647601 A1 | 03-01-2008 |
| | | CN 101479241 A | 08-07-2009 |
| | | EP 2041085 A1 | 01-04-2009 |
| | | JP 2009544581 A | 17-12-2009 |
| | | KR 20090021165 A | 27-02-2009 |
| | | US 2008064695 A1 | 13-03-2008 |
| | | US 2010216748 A1 | 26-08-2010 |
| | | WO 2008002974 A1 | 03-01-2008 |
| | | ZA 200808757 B | 31-03-2010 |
| WO 2016014847 A1 | 28-01-2016 | EP 3180086 A1 | 21-06-2017 |
| | | US 2017217984 A1 | 03-08-2017 |
| | | WO 2016014847 A1 | 28-01-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SPARKS, T.C. ; WATSON, G.B. ; LOSO, M.R. ; GENG, C. ; BABCOCK, J.M. ; THOMAS, J.D.** Sulfoxaflor and the sulfoximine insecticides: Chemistry, mode of action and basis for efficacy on resistant insects. *Pesticide Biochemistry and Physiology,* 2013, vol. 107 (1), 1-7
- **NAUEN, R. ; JESCHKE, P. ; VELTEN, R. ; BECK, M.E. ; EBBINGHAUS-KINTSCHER, U. ; THIELERT, W. ; WOELFEL, K. ; HAAS, M. ; KUNZ, K. ; RAUPACH, G.** Flupyradifurone: a brief profile of a new butenolide insecticide. *Pest Management Science,* 2015, vol. 71 (6), 850-862
- **SUPURAN, C. T. ; INNOCENTI, A. ; MASTROLORENZO, A. ; SCOZZAFAVA, A.** Antiviral sulfonamide derivatives. *Mini-Reviews in Medicinal Chemistry,* 2004, vol. 4 (2), 189-200
- **CAMPBELL, J. W. ; CABRERA, A. R. ; STANLEY-STAHR, C. ; ELLIS, J. D.** An Evaluation of the Honey Bee (Hymenoptera: Apidae) Safety Profile of a New Systemic Insecticide, Flupyradifurone, Under Field Conditions in Florida. *Journal of Economic Entomology,* 2016, vol. 109 (5), 1967-1972
- **SCOZZAFAVA, A. ; OWA, T. ; MASTROLORENZO, A. ; SUPURAN, C. T.** Anticancer and antiviral sulfonamides. *Current Medicinal Chemistry,* 2003, vol. 10 (11), 925-953
- **MCELVAIN, S. M. ; GOESE, M. A.** The Sulfonation of Pyridine and the Picolines. *Journal of the American Chemical Society,* 1943, vol. 65 (11), 2233-2236
- **BRAND, J. C. D.** Aromatic sulphonation. Part II. Kinetics of sulphonation in fuming sulphuric acid. *Journal of the Chemical Society (Resumed),* 1950, vol. 207 (0), 1004-1017
- **THOMAS, K. ; JERCHEL, D.** Neuere Methoden der präparativen organischen Chemie II. 12. Die Einführung von Substituenten in den Pyridin-Ring. *Angewandte Chemie,* 1958, vol. 70 (24), 719-737
- **OWA, T. ; YOSHINO, H. ; OKAUCHI, T. ; OKABE, T. ; OZAWA, Y. ; HATA SUGI, N. ; YOSHIMATSU, K. ; NAGASU, T. ; KOYANAGI, N. ; KITOH, K.** Synthesis and biological evaluation of N-(7-indolyl)-3-pyridinesulfonamide derivatives as potent antitumor agents. *Bioorganic & Medicinal Chemistry Letters,* 2002, vol. 12 (16), 2097-2100
- **MASLANKIEWICZ, A. ; MARCINIEC, K. ; PAWLOWSKI, M. ; ZAJDEL, P.** From haloquinolines and halopyridines to quinoline- and pyridinesulfonyl chlorides and sulfonamides. *Heterocycles,* 2007, vol. 71 (9), 1975-1990
- **ZAJDEL, P. ; MARCINIEC, K. ; MASLANKIEWICZ, A. ; GRYCHOWSKA, K. ; SATALA, G. ; DUSZYNSKA, B. ; LENDA, T. ; SIWEK, A. ; NOWAK, G. ; PARTYKA, A.** Antidepressant and antipsychotic activity of new quinoline- and isoquinoline-sulfonamide analogs of aripiprazole targeting serotonin 5-HT1A/5-HT2A/5-HT7 and dopamine D-2/D-3 receptors. *European Journal of Medicinal Chemistry,* 2013, vol. 60, 42-50
- **PU, Y. M. ; CHRISTESEN, A. ; KU, Y. Y.** A simple and highly effective oxidative chlorination protocol for the preparation of arenesulfonyl chlorides. *Tetrahedron Letters,* 2010, vol. 51 (2), 418-421
- **PHILIP J. HOGAN ; BRIAN G. COX.** Aqueous Process Chemistry: The Preparation of Aryl Sulfonyl Chlorides. *Org. Process Res. Dev.,* 2009, vol. 13 (5), 875-879
- **TALLEY, T. T. ; HAREL, M. ; HIBBS, R. E. ; RADIC, Z. ; TOMIZAWA, M. ; CASIDA, J. E. ; TAYLOR, P.** Atomic interactions of neonicotinoid agonists with AChBP: Molecular recognition of the distinctive electronegative pharmacophore. *Proceedings of the National Academy of Sciences of the United States of America,* 2008, vol. 105 (21), 7606-7611
- **IRWIN, J. J. ; SHOICHET, B. K.** ZINC - A free database of commercially available compounds for virtual screening. *Journal of Chemical Information and Modeling,* 2005, vol. 45 (1), 177-182
- **HAWKINS, P.C.D. ; SKILLMAN, A.G. ; NICHOLLS, A.** Comparison of Shape-Matching and Docking as Virtual Screening Tools. *Journal of Medicinal Chemistry,* 2007, vol. 50, 74-82
- **FRIESNER, R. A. ; BANKS, J. L. ; MURPHY, R. B. ; HALGREN, T. A. ; KLICIC, J. J. ; MAINZ, D. T. ; REPASKY, M. P. ; KNOLL, E. H. ; SHELLEY, M. ; PERRY, J. K.** Glide: a new approach for rapid, accurate docking and scoring. 1. Method and assessment of docking accuracy. *Journal of medicinal chemistry,* 2004, vol. 47 (7), 1739-1749

- **SELVAM, B. ; GRATON, J. ; LAURENT, A. D. ; ALAMIDDINE, Z. ; MATHE-ALLAINMAT, M. ; LEBRETON, J. ; COQUERET, O. ; OLIVIER, C. ; THANY, S. H. ; LE QUESTEL, J.-Y.** Imidacloprid and thiacloprid neonicotinoids bind more favourably to cockroach than to honeybee alpha 6 nicotinic acetylcholine receptor: Insights from computational studies. *Journal of Molecular Graphics & Modelling,* 2015, vol. 55, 1-12
- Schrödinger Release 2014-1: LigPrep. LLC, 2014
- Schrödinger Release. LLC, January 2014
- **FRIESNER, R.A. ; MURPHY, R.B. ; REPASKY, M.P. ; FRYE, L.L. ; GREENWOOD, J.R. ; HALGREN, T.A. ; SANSCHAGRIN, P. C. ; MAINZ, D. T.** Extra precision glide: docking and scoring incorporating a model of hydrophobic enclosure for protein-ligand complexes. *Journal of medicinal chemistry,* 2006, vol. 49, 6177-96
- **JESCHKE, P. ; NAUEN, R. ; BECK, M. E.** Nicotinic Acetylcholine Receptor Agonists: A Milestone for Modern Crop Protection. *Angewandte Chemie-International Edition,* 2013, vol. 52 (36), 9464-9485
- **BLUM, A. P. ; LESTER, H. A. ; DOUGHERTY, D. A.** Nicotinic pharmacophore: The pyridine N of nicotine and carbonyl of acetylcholine hydrogen bond across a subunit interface to a backbone NH. *Proceedings of the National Academy of Sciences of the United States of America,* 2010, vol. 107 (30), 13206-13211
- **XIAO, Y. ; HAMMOND, P. S. ; MAZUROV, A. A. ; YOHANNES, D.** Multiple Interaction Regions in the Orthosteric Ligand Binding Domain of the alpha 7 Neuronal Nicotinic Acetylcholine Receptor. *Journal of Chemical Information and Modeling,* 2012, vol. 52 (11), 3064-3073
- **AMIRI, S. ; SANSOM, M. S. P. ; BIGGIN, P. C.** Molecular dynamics studies of AChBP with nicotine and carbamylcholine: the role of water in the binding pocket. *Protein Engineering Design & Selection,* 2007, vol. 20 (7), 353-359
- **BLUM, A. P. ; GLEITSMAN, K. R. ; LESTER, H. A. ; DOUGHERTY, D. A.** Evidence for an Extended Hydrogen Bond Network in the Binding Site of the Nicotinic Receptor Role of the Vicinal Disulfide of the Alpha 1 Subunit. *Journal of Biological Chemistry,* 2011, vol. 286 (37), 32251-32258
- **PUSKAR, N. L. ; XIU, X. ; LESTER, H. A. ; DOUGHERTY, D. A.** Two Neuronal Nicotinic Acetylcholine Receptors, alpha 4 beta 4 and alpha 7, Show Differential Agonist Binding Modes. *Journal of Biological Chemistry,* 2011, vol. 286 (16), 14618-14627
- **XIU, X. ; PUSKAR, N. L. ; SHANATA, J. A. P. ; LESTER, H. A. ; DOUGHERTY, D. A.** Nicotine binding to brain receptors requires a strong cation-$\pi$ interaction. *Nature (London, U. K.) Nature (London, United Kingdom),* 2009, vol. 458 (7237), 534-537
- **SINE, S. M.** End-plate acetylcholine receptor: structure, mechanism, pharmacology, and disease. *Physiological Reviews,* 2012, vol. 92 (3), 1189-1234
- **RUCKTOOA, P. ; SMIT, A. B. ; SIXMA, T. K.** Insight in nAChR subtype selectivity from AChBP crystal structures. *Biochemical Pharmacology,* 2009, vol. 78 (7), 777-787
- **CELIE, P. H. N. ; VAN ROSSUM-FIKKERT, S. E. ; VAN DIJK, W. J. ; BREJC, K. ; SMIT, A. B. ; SIXMA, T. K.** Nicotine and carbamylcholine binding to nicotinic acetylcholine receptors as studied in AChBP crystal structures. *Neuron,* 2004, vol. 41 (6), 907-914
- Molecular recognition of thiaclopride by Aplysia californica AChBP: new insights from a computational investigation. **ALAMIDDINE, Z. ; SELVAM, B. ; CERON-CARRASCO, J. P. ; MATHE-ALLAINMAT, M. ; LEBRETON, J. ; THANY, S. H. ; LAURENT, A. D. ; GRATON, J. ; LE QUESTEL, J.-Y.** J. Comput.-Aided Mol. Des. American Chemical Society (ACS), 2015, vol. 29, 1151-1167
- Insights into a highly conserved network of hydrogen bonds in the agonist binding site of nicotinic acetylcholine receptors: a structural and theoretical study. **ATKINSON, A. ; GRATON, J. ; LE, Q. J.-Y.** Proteins. U.S. National Library of Medicine, 2014, vol. 82, 2303-17
- New Insights on the Molecular Recognition of Imidacloprid with Aplysia californica AChBP: A Computational Study. **CERON-CARRASCO, J. P. ; JACQUEMIN, D. ; GRATON, J. ; THANY, S. ; LE QUESTEL, J.-Y.** Journal of Physical Chemistry B. American Chemical Society (ACS), 2013, vol. 117, 3944-3953
- **SPARKS, T. C. ; WATSON, G. B. ; LOSO, M. R. ; GENG, C. ; BABCOCK, J. M. ; THOMAS, J. D.** Sulfoxaflor and the sulfoximine insecticides: Chemistry, mode of action and basis for efficacy on resistant insects. *Pesticide Biochemistry and Physiology,* 2013, vol. 107 (1), 1-7
- **WANG, N. X. ; WATSON, G. B. ; LOSO, M. R. ; SPARKS, T. C.** Molecular modeling of sulfoxaflor and neonicotinoid binding in insect nicotinic acetylcholine receptors: impact of the Myzus $\beta$1 R81T mutation. *Pest Manage. Sci.,* 2016, vol. 72, 1467-1474
- **CUTLER, P. ; SLATER, R. ; EDMUNDS, A. J. F. ; MAIENFISCH, P. ; HALL, R. G. ; EARLEY, F. G. P. ; PITTERNA, T. ; PAL, S. ; PAUL, V.-L. ; GOODCHILD, J.** Investigating the mode of action of sulfoxaflor: a fourth-generation neonicotinoid. *Pest Management Science,* 2013, vol. 69 (5), 607-619
- **BAHDE, R. J. ; RYCHNOVSKY, S. D.** Cyclization via Carbolithiation of $\alpha$-Amino Alkyllithium Reagents. *Organic Letters,* 2008, vol. 10 (18), 4017-4020

- **DE KIMPE, N. G. ; STEVENS, C. V. ; KEPPENS, M. A.** Synthesis of 2-acetyl-1-pyrroline, the principal rice flavor component. *Journal of Agricultural and Food Chemistry,* 1993, vol. 41 (9), 1458-1461
- **LIU, X.-W. ; LE, T. N. ; LU, Y. ; XIAO, Y. ; MA, J. ; LI, X.** An efficient synthesis of chiral phosphinyl oxide pyrrolidines and their application to asymmetric direct aldol reactions. *Organic & Biomolecular Chemistry,* 2008, vol. 6 (21), 3997-4003
- **KÖHLER, V. ; BAILEY, K. R. ; ZNABET, A. ; RAFTERY, J. ; HELLIWELL, M. ; TURNER, N. J.** Enantioselective Biocatalytic Oxidative Desymmetrization of Substituted Pyrrolidines. *Angewandte Chemie International Edition,* 2010, vol. 49 (12), 2182-2184
- **JI, X. ; SU, M. ; WANG, J. ; DENG, G. ; DENG, S. ; LI, Z. ; TANG, C. ; LI, J. ; LI, J. ; ZHAO, L.** Design, synthesis and biological evaluation of hetero-aromatic moieties substituted pyrrole-2-carbonitrile derivatives as dipeptidyl peptidase IV inhibitors. *European Journal of Medicinal Chemistry,* 2014, vol. 75, 111-122
- **CALLEC, J. J. ; SATTELLE, D. B.** A simple technique for monitoring the synaptic actions of pharmacological agents. *J Exp Biol,* 1973, vol. 59 (3), 725-38
- Central synaptic actions of pharmacological agents in insects: oil-gap and mannitol-gap studies. **CALLEC, J. J. ; SATTELLE, D. B. ; HUE, B. ; PELHATE, M.** Neurotox. Plenum Press, 1980, vol. 79, 93-100
- Central synaptic actions of pharmacological agents in insects: oil-gap and mannitol-gap studies. **CALLEC, J.J. ; SATTELLE, D.B. ; HUE, B. ; PELHATE, M.** Neurotox. Plenum Press, 1980, vol. 79, 93-100
- **BUCKINGHAM, S. ; LAPIED, B. ; CORRONC, H. ; SATTELLE, F.** Imidacloprid actions on insect neuronal acetylcholine receptors. *J Exp Biol,* 1997, vol. 200, 2685-92
- **THANY, S. H.** Agonist actions of clothianidin on synaptic and extrasynaptic nicotinic acetylcholine receptors expressed on cockroach sixth abdominal ganglion. *Neurotoxicology,* 2009, vol. 30 (6), 1045-52
- **BUCKINGHAM, S.D. ; LAPIED, B. ; LECORRONC, H. ; GROLLEAU, F. ; SATTELLE, D.B.** Imidacloprid actions on insect neuronal acetylcholine receptors. *J. Exp. Biol.,* 1997, vol. 200 (21), 2685-2692
- **THANY, S. H.** Agonist actions of clothianidin on synaptic and extrasynaptic nicotinic acetylcholine receptors expressed on cockroach sixth abdominal ganglion. *Neurotoxicology,* 2009, vol. 30 (6), 1045-1052
- **NAUEN, R. ; EBBINGHAUS-KINTSCHER, U. ; SCHMUCK, R.** Toxicity and nicotinic acetylcholine receptor interaction of imidacloprid and its metabolites in Apis mellifera (Hymenoptera : Apidae). *Pest Management Science,* 2001, vol. 57 (7), 577-586
- **IWASA, T. ; MOTOYAMA, N. ; AMBROSE, J. T. ; ROE, R. M.** Mechanism for the differential toxicity of neonicotinoid insecticides in the honey bee, Apis mellifera. *Crop Protection,* 2004, vol. 23 (5), 371-378
- **STARK, J. D. ; JEPSON, P. C. ; MAYER, D. F.** Limitations to use of topical toxicity data for predictions of pesticide side-effects in the field. *Journal of Economic Entomology,* 1995, vol. 88 (5), 1081-1088
- Bcpc; Bcpc, Thiacloprid, a novel neonicotinoid insecticide for foliar application. **ELBERT, A. ; ERDELEN, C. ; KUHNHOLD, J. ; NAUEN, R. ; SCHMIDT, H. U. ; HATTORI, Y.** Bcpc Conference: Pests & Diseases 2000, Vols 1-3, Proceedings. 2000, vol. 1-3, 21-26